# EUROPÄISCHE PATENTSCHRIFT

(11) **EP 2 611 869 B1**
(45) Veröffentlichungstag und Bekanntmachung des Hinweises auf die Patenterteilung: **01.10.2014**
(21) Anmeldenummer: 11743449.8
(22) Anmeldetag: 05.08.2011
(51) Int. Cl.: C09B 31/00, C07D 407/12

(54) **FARBSTOFF-ASCORBINSÄUREDERIVATE**
DYE-ASCORBIC ACID DERIVATIVES
DÉRIVÉS COLORANTS DE L'ACIDE ASCORBIQUE

(30) Priorität: 04.09.2010 DE 102010044381
(43) Veröffentlichungstag der Anmeldung: 10.07.2013
(73) Patentinhaber: Merck Patent GmbH, 64293 Darmstadt (DE)
(72) Erfinder: RUDOLPH, Thomas, 64291 Darmstadt (DE); BUEHLE, Philipp, 64291 Darmstadt (DE)
(86) Internationale Anmeldenummer: PCT/EP2011/003951
(87) Internationale Veröffentlichungsnummer: WO 2012/028245

(56) Entgegenhaltungen:
- JP-A- 2008 260 900

## Beschreibung

Gegenstand der Erfindung sind spezielle Farbstoff-Ascorbinsäurederivate der Formel I, wie nachfolgend beschrieben, und deren Verwendung als Farbstoff zur Färbung einer Matrix, wie zum Beispiel Haut, Haare, Nägel oder Textilien, sowie ein Verfahren zu deren Herstellung. Des Weiteren betrifft die Erfindung ein Verfahren zum Färben einer Matrix mit zumindest einem speziellen Farbstoff-Ascorbinsäurederivat. Ein weiterer Gegenstand der Erfindung ist eine Zubereitung, enthaltend zumindest ein spezielles Farbstoff-Ascorbinsäurederivat, sowie ein Verfahren zur Herstellung einer solchen Zubereitung.

Die JP2008/260900 offenbart Farbstoffe auf Basis von Azoverbindungen, welche Ascorbinsäure als stabilisierende Gruppe enthalten.

Derzeit ist zum Färben einer Matrix, wie zum Beispiel Haut, Haare, Nägel oder Textilien eine Vielzahl von Direktfarbstoffen bekannt. Dabei assoziieren sich die Farbstoffe an die Matrix und/oder bilden kovalente chemische Bindungen zu der Matrix aus. Diese Anbindung der Farbstoffmoleküle an die Matrix kann in unterschiedlicher Art und Weise stattfinden und zu einem unterschiedlichen Ergebnis hinsichtlich des Anbindungscharakters führen. Deshalb zeichnen sich die Farbstoffe auch durch eine unterschiedlich starke Anbindungsfähigkeit an die jeweilige Matrix aus.

Oft ist bei derzeit gebräuchlichen Farbstoffen eben diese Anbindungsfähigkeit schwach ausgeprägt, sodass der Farbstoff schnell durch zum Beispiel Schweiß oder Wasser ausgewaschen werden kann. Aufgrund der geringen Anbindungsfähigkeit des Farbstoffes an die jeweilige Matrix ist zudem die Ergiebigkeit des Farbstoffes im Färbeprozess gering und damit einhergehend kann die Intensität der Färbung von Matrices gering ausgeprägt sein. Bei Verwendung von, insbesondere synthetischen, Farbstoffen kann, insbesondere im humanen Anwendungsbereich, zudem eine geringe Verträglichkeit vorliegen.

Somit besteht weiterhin ein Bedarf an, unter anderem, verträglichen und insbesondere hautverträglichen, Farbstoffen, die eine gute Anbindungsfähigkeit der Farbstoffmoleküle an die jeweilige Matrix aufweisen, so dass die jeweilige Matrix dauerhaft gefärbt werden kann.

Demzufolge beschäftigt sich die vorliegende Erfindung mit dem Problem, verbesserte oder zumindest alternative Farbstoffe zum Färben von Matrices anzugeben, die sich insbesondere durch eine verbessertes Färbeverhalten auszeichnen und die eine positive Auswirkung auf die Feuchtigkeit der Matrix ausüben, was bei einer Anwendung auf Haare zu einer erhöhten Haarelastizität führt.

Erfindungsgemäß wird dieses Problem durch die Gegenstände der unabhängigen Ansprüche gelöst. Vorteilhafte Ausführungsformen sind Gegenstand der abhängigen Ansprüche.

Überraschenderweise wurde nun festgestellt, dass sich spezielle Farbstoff-Ascorbinsäurederivate, insbesondere die Verbindungen der Formel I, in hervorragender Weise zum Färben von Matrices eignen. Dabei kann die Ascorbinsäure bzw. die Dehydroascorbinsäure in dem jeweiligen Farbstoff-Ascorbinsäurederivat in L-Form oder D-Form oder in einer Mischung beider isomerer Formen vorliegen. Bevorzugt ist die Ascorbinsäure bzw. die Dehydroascorbinsäure in dem jeweiligen Farbstoff-Ascorbinsäurederivat in L-Form.

Fortfolgend steht jeweils unabhängig voneinander:
Alk¹ für eine geradkettige oder verzweigte C₁- bis C₂₀-Alkylgruppe oder für eine geradkettige oder verzweigte C₂- bis C₂₀-Alkenylgruppe, die mehrere Doppelbindungen aufweisen kann, wobei zumindest ein C-Atom oder mehrere nicht benachbarte C-Atome der C₁- bis C₂₀-Alkyl- oder C₂- bis C₂₀-Alkenylgruppe durch O ersetzt sein kann/können, wobei die C₁- bis C₂₀-Alkyl- oder C₂- bis C₂₀-Alkenylgruppe zumindest ein an ein primäres oder sekundäres C-Atom angebundenes OH, F, Cl, Br oder I aufweisen kann;
Alk² für eine geradkettige oder verzweigte C₁- bis C₂₀-Alkylgruppe;
Alk³ für eine geradkettige oder verzweigte C₁- bis C₈-Alkylgruppe;
Cyc für eine C₃- bis C₈-Cycloalkyl-Gruppe, die zumindest eine Doppelbindung aufweisen kann, und/oder in der zumindest ein CH₂ durch O oder NH ersetzt sein kann;
Arl für eine unsubstituierte, einfach- oder mehrfachsubstituierte C₆- bis C₂₀-Arylgruppe, wobei als Substituenten OH, N(Alk¹)₂, N(Alk¹)₃An⁻, Alk¹, OAlk¹, Phenyl, Biphenyl, Naphthyl und Anthryl umfasst sind;

Dabei umfasst eine:
C₁-C₄-Alkylgruppe Methyl, Ethyl, Propyl-, iso-Propyl, Butyl, x-Methylpropyl (x=1;2) und tert.-Butyl;
   C₁-C₈-Alkylgruppe die Substituenten der C₁-C₄-Alkylgruppe und Pentyl, Hexyl, Heptyl, Oktyl, x-Methylbutyl (x=1;2;3), x-Methylpentyl (x=1;2;3;4), x-Methylhexyl (x=1;2;3;4;5), x-Ethylpentyl (x=1;2;3) und x-Ethylhexyl (x=1;2;3;4);
   C₁-C₂₀-Alkylgruppe die Substituenten der C₁-C₈-Alkylgruppe und Nonanyl, Decanyl, Undecanyl, Dodecanyl, Tridecanyl, Tetradecanyl, Pentadecanyl, Hexadecanyl, Heptadecanyl, Oktadecanyl, Nonadecanyl und Eicosanyl;
C₂-C₄-Alkenylgruppe Ethenyl, x-Propenyl (x=1;2), x-Butenyl (x=1;2;3) und Butadienyl;
   C₂-C₈-Alkenylgruppe die Substituenten der C₂-C₄-Alkenylgruppe und x-Pentenyl (x=1;2;3;4), x-Hexenyl (x=1;2;3;4;5), x-Heptenyl (x=1;2;3;4;5;6) und x-Oktenyl (x=1;2;3;4;5;6;7);
   C₂-C₂₀-Alkenylgruppe die Substituenten der C₂-C₁₂-Alkenylgruppe und x-Nonenyl (x=1;2;3;4;5;6;7;8), x-Decenyl (x=1;2;3;4;5;6;7;8;9), x-Undecenyl (x=1;2;3;4;5;6;7;8;9;10), x-Dodecenyl (x=1;2;3;4;5;6;7;8;9;10;11), 9-Oktadecenyl, 9,12-Oktadecadienyl, 9,12,15-Oktadecatrienyl, 10-Nonadecenyl, 10,13-Nonadecadienyl, 10,13,16-Nonadecatrienyl, 11-Eicosenyl, 11,14-Eicosadienyl und 11,14,17-Eicosatrienyl; C₅-C₆-Cycloalkylgruppe Cyclopentyl, x-Cyclopentenyl (x=1;2;3), x,y-Cyclopentadeinyl (x,y=1,3;1,4;2,4), Cyclohexyl, x-Cyclohexenyl (x=1;2;3), x,y-Cyclohexadienyl (x,y=1,3;1,4;2,4;2,5), x-Tetrahydrofuranyl (x=2;3), x-(2,5-Dihydrofuranyl) (x=2;3), x-Tetrahydropyrrolyl (x=1;2;3), x-(2,5-Dihydropyrrolyl) (x=1,2;3), x-(4,5-Dihydrooxazolyl) (x=2;4;5), x-Oxazolidinyl (x=2;3;4;5), x-Piperidinyl (x=2;3;4;5), x-Pyranyl (x=2;3;4;5), x-(4,5-Dihydropyranyl) (x=2;3;4;5), x-Piperazinyl (x=2;3), x-(1,3-Dioxanyl) (x=2;4;5;6) und x-(1,4-Dioxanyl) (x=2;3;5;6), x-(Tetrahydro-1,2-oxazinyl) (x=2;3;4;5;6), x-(Tetrahydro-1,3-oxazinyl) (x=2;3;4;5;6) und x-(Tetrahydro-1,4-oxazinyl) (x=2;3;4;5;6); C₃-C₈-Cycloalkylgruppe die Substituenten der C₅-C₆-Cycloalkylgruppe und Cyclopropyl, x-Cyclopropenyl (x=1;2), Cyclobutanyl, x-Cyclobutenyl (x=1;2;3), Cycloheptyl, Cyclooktyl, x-Methylcylclohexyl (x=2;3;4), x-Ethylcyclohexyl (x=2;3;4) und x,y-Dimethylcyclohexyl (x,y=2,3; 2,4);
C₆-C₁₂-Arylgruppe Phenyl, Naphthyl und Biphenylyl, C₆-C₁₅-Arylgruppe, die Substituenten der Cₑ-C₁₂-Arylgruppe und Anthryl, und Phenanthryl; C₆-C₂₀-Arylgruppe, die Substituenten der C₆-C₁₅-Arylgruppe und Pyrenyl, Chrysenyl Naphthacenyl und Picenyl;
einfach oder mehrfach substituierte C₆-C₁₂-Arylgruppe, x-Methylphenyl (x=o,m,p), x-Ethylphenyl (x=o,m,p), x-Propylphenyl (x=o,m,p), x-Isopropylphenyl (x=o,m,p), x-tert.-Butylphenyl (x=o,m,p), x-Nitrophenyl (x=o,m,p), x-Methoxyphenyl (x=o,m,p), x-Ethoxyphenyl (x=o,m,p), (x=o,m,p), x,y-Dimethylphenyl (x=2,3;2,4;2,5;2,6;3,4;3,5), x,y-Dihydroxyphenyl (x=2,3;2,4;2,5;2,6;3,4;3,5), x,y-Dimethoxyphenyl (x=2,3;2,4;2,5;2,6;3,4;3,5), 3,4,5-Trimethoxyphenyl und 2,4,5-Trimethylphenyl,
   wobei als weitere Substituenten OH, N(Alk¹)₂, N(Alk¹)₃An⁻, Alk¹, OAlk¹, Phenyl, Biphenyl, Naphthyl und Anthryl auftreten können;
   einfach oder mehrfach substituierte C₆-C₁₅-Arylgruppe, die Substituenten der einfach oder mehrfach substituierten C₆-C₁₂-Arylgruppe;
   einfach oder mehrfach substituierte C₆-C₂₀-Arylgruppe, die Substituenten der einfach oder mehrfach substituierten C₆-C₁₅-Arylgruppe.

Ein Gegenstand der Erfindung sind somit Verbindungen der Formel I 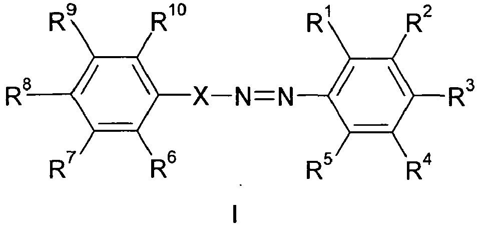 wobei X steht für eine Einfachbindung oder einen Substituenten der Formel IIa oder IIb 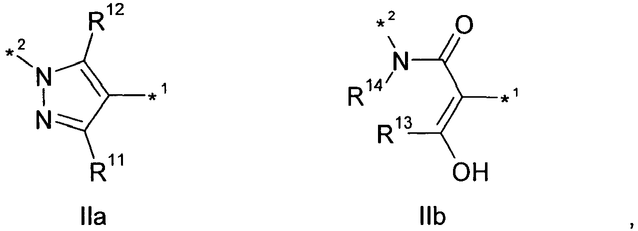 wobei *¹ zur N=N orientiert ist,
wobei genau einer der Substituenten R¹ bis R¹⁴ steht für einen Substituenten der Formel IIIa, IIIb oder IIIc 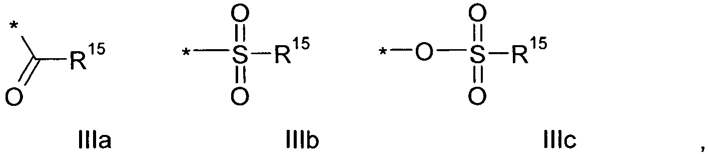 wobei R¹⁵ jeweils unabhängig voneinander steht für einen Ascorbinsäurerest der Formel IVa oder IVb oder einen Dehydroascorbinsäurerest der Formel IVc oder IVd 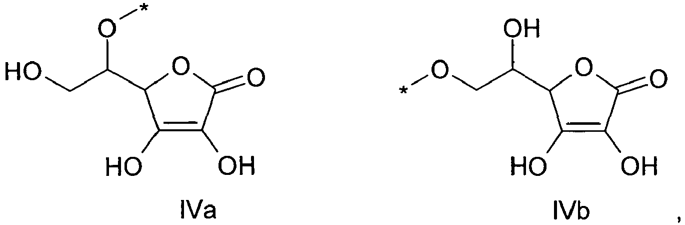 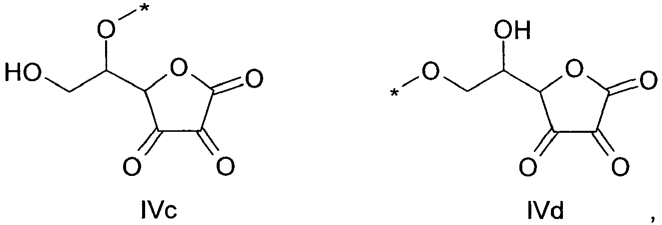 wobei die verbleibenden Substituenten R¹ bis R¹⁴ jeweils unabhängig voneinander stehen
für H, OH, NH₂, NO₂, F, Cl, Br, I, CF₃, OCF₃, O(C=O)-CH₂-NH₂, Alk¹, OAlk¹, NHAlk¹, NAlk¹₂, Cyc, (NAlk¹₃)⁺ₓAn^{x-}, (SO₂)NH₂, (SO₂)NHAlk¹, O(SO₂)OAlk¹, O(SO₂)OArl, (C=O)OAlk¹, (C=O)OArl oder Arl,
wobei Alk¹ jeweils unabhängig voneinander steht
für eine geradkettige oder verzweigte C₁- bis C₂₀-Alkylgruppe oder für eine geradkettige oder verzweigte C₂- bis C₂₀-Alkenylgruppe, die mehrere Doppelbindungen aufweisen kann, wobei zumindest ein C-Atom oder mehrere nicht benachbarte C-Atome der C₁- bis C₂₀-Alkyl- oder C₂- bis C₂₀-Alkenylgruppe durch O ersetzt sein kann/können, wobei die C₁- bis C₂₀-Alkyl- oder C₂- bis C₂₀-Alkenylgruppe zumindest ein an ein primäres oder sekundäres C-Atom angebundenes OH, F, Cl, Br oder I aufweisen kann, wobei Arl jeweils unabhängig voneinander steht
für eine unsubstituierte, einfach- oder mehrfachsubstituierte C₆- bis C₂₀-Arylgruppe,
wobei Cyc steht für eine C₃- bis C₈-Cycloalkyl-Gruppe, die zumindest eine Doppelbindung aufweisen kann und/oder in der zumindest ein CH₂ durch O oder NH ersetzt sein kann,
wobei An^{x-} jeweils unabhängig voneinander steht für ein Anion der Ladung 1≤ x ≤ 3 und/oder ihrer Tautomere, Stereoisomere, einschließlich deren Mischungen in allen Verhältnissen.

Als Anion An^{x-} kommen alle Anionen in Frage, die für eine kosmetische Anwendung verträglich sind. Beispielhafte Anionen sind Anionen der physiologisch unbedenklichen Säuren aus der Gruppe Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure.

Eine Verbindung der Formel I enthaltend mindestens eine Funktionalität N(Alk¹)₂, kann mit einer Säure in das zugehörige Säureadditionssalz mit der Gruppierung N(Alk¹)₃An^{x-} überführt werden, beispielsweise durch Umsetzung äquivalenter Mengen dieser Verbindung der Formel I und der Säure in einem inerten Lösungsmittel wie Ethanol und anschließendes Eindampfen. Die Säure wird ausgewählt wie zuvor beschrieben.

Durch Derivatisierung von speziellen Azoverbindungen der Formel V, wie nachfolgend beschrieben, mit Ascorbinsäure bzw. Dehydroascorbinsäure ist das Färbeverhalten solcher Verbindungen der Formel I im Vergleich zu den Azoverbindungen der Formel V selbst überraschend deutlich verbessert.

Dabei stellt die Dehydroascorbinsäure der Formel IX eine oxidierte Form der Ascorbinsäure der Formel VIII dar, die im menschlichen Organismus wieder zu Ascorbinsäure reduziert werden kann.

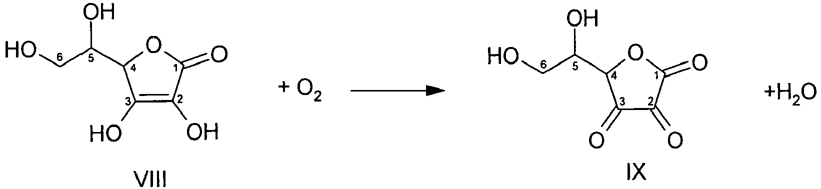

Die Nummerierung der Positionen der Ascorbinsäure der Formel VIII, sowie der Dehydroascorbinsäure der Formel IX gilt dabei wie in obigem Reaktionsschema dargestellt.

Da Azoverbindungen eine Azo-Hydrazo-Tautomerie aufweisen und bei dementsprechenden Umgebungsbedingungen zu einem vorbestimmten Prozentsatz sowohl in der Azoform, als auch in der Hydrazo-Form vorliegen, ist im Sinne der Erfindung unter einer Verbindung der Formel I die in der Formel I gezeigte Azoform als auch die nicht dargestellte Hydrazoform zu verstehen.

Bevorzugt sind Verbindungen der Formel I, wobei R¹⁵ jeweils unabhängig voneinander steht für einen L-Ascorbinsäurerest der Formel Iva-1 oder IVb-1 oder einen L-Dehydroascorbinsäurerest der Formel IVc-1 oder IVd-1.

Bevorzugte Verbindungen der Formel I sind daher Verbindungen der Formel I 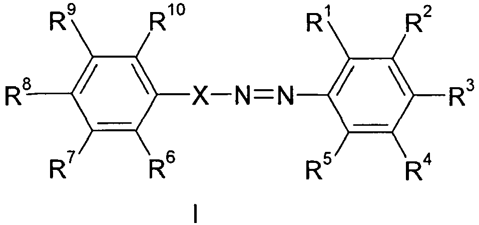 wobei X steht für eine Einfachbindung oder einen Substituenten der Formel IIa oder IIb 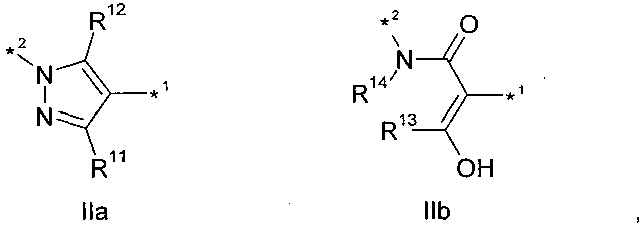 wobei *¹ zur N=N orientiert ist,
wobei genau einer der Substituenten R¹ bis R¹⁴ steht für einen Substituenten der Formel IIIa, IIIb oder IIIc 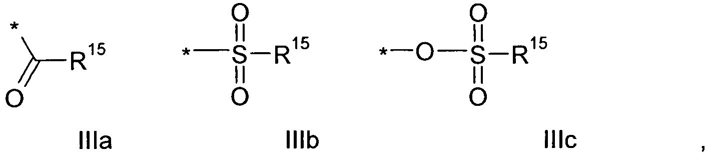 wobei R¹⁵ jeweils unabhängig voneinander steht für einen L-Ascorbinsäurerest der Formel Iva-1 oder IVb-1 oder einen Dehydroascorbinsäurerest der Formel IVc-1 oder IVd-1 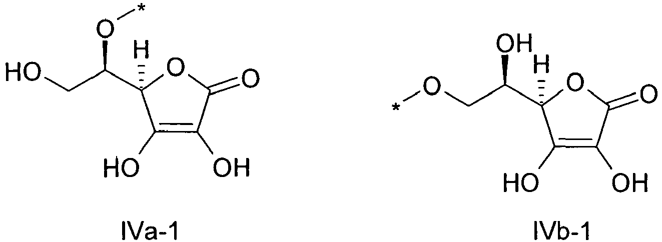 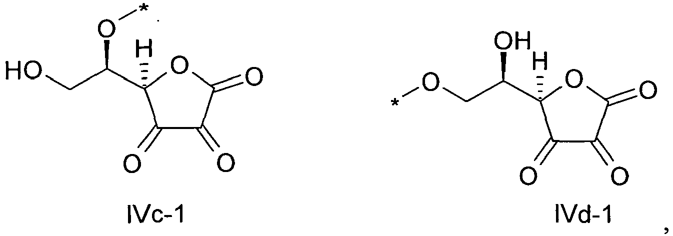 wobei die verbleibenden Substituenten R¹ bis R¹⁴ jeweils unabhängig voneinander stehen
für H, OH, NH₂, NO₂, F, Cl, Br, I, CF₃, OCF₃, O(C=O)-CH₂-NH₂, Alk¹, OAlk¹, NHAlk¹, NAlk¹₂, Cyc, (NAlk¹₃)⁺ₓAn^{x-}, (SO₂)NH₂, (SO₂)NHAlk¹, O(SO₂)OAlk¹, O(SO₂)OArl, (C=O)OAlk¹, (C=0)0Arl oder Arl,
wobei Alk¹ jeweils unabhängig voneinander steht
für eine geradkettige oder verzweigte C₁- bis C₂₀-Alkylgruppe oder für eine geradkettige oder verzweigte C₂- bis C₂₀-Alkenylgruppe, die mehrere Doppelbindungen aufweisen kann, wobei zumindest ein C-Atom oder mehrere nicht benachbarte C-Atome der C₁- bis C₂₀-Alkyl- oder C₂- bis C₂₀-Alkenylgruppe durch O ersetzt sein kann/können, wobei die C₁- bis C₂₀-Alkyl- oder C₂- bis C₂₀-Alkenylgruppe zumindest ein an ein primäres oder sekundäres C-Atom angebundenes OH, F, Cl, Br oder I aufweisen kann,
wobei Arl jeweils unabhängig voneinander steht
für eine unsubstituierte, einfach- oder mehrfachsubstituierte C₆- bis C₂₀-Arylgruppe,
wobei Cyc steht für eine C₃- bis C₈-Cycloalkyl-Gruppe, die zumindest eine Doppelbindung aufweisen kann und/oder in der zumindest ein CH₂ durch O oder NH ersetzt sein kann,
wobei An^{x-} jeweils unabhängig voneinander steht für ein Anion der Ladung 1≤x≤3.

Bevorzugt sind Verbindungen der Formel I, wobei die verbleibenden Substituenten R¹ bis R¹⁴ jeweils unabhängig voneinander stehen für H, OH, NH₂, NO₂, F, Cl, Br, I, CF₃, OCF₃, Alk¹, OAlk¹, NHAlk¹, NAlk¹₂, (NAlk¹₃)⁺ₓAn^{x-}, (SO₂)NH₂, (SO₂)NHAlk¹, (C=O)OAlk¹, (C=O)OArl oder Arl. Besonders bevorzugt sind Verbindungen der Formel I, wobei die verbleibenden Substituenten R¹ bis R¹⁴ jeweils unabhängig voneinander stehen für H, OH, N(Alk¹)₂, Alk¹, N(Alk¹)₃An⁻ oder Arl.
Bevorzugt ist es, wenn zumindest einer der verbleibenden Substituenten R¹ bis R¹⁴ steht für OH, Alk¹, N(Alk¹)₂, (NAlk¹₃)⁺ₓAn^{x-} oder Arl und die übrigen verbleibenden Substituenten stehen für H.

Ganz besonders bevorzugt sind Verbindungen der Formel I, wobei die verbleibenden Substituenten R¹ bis R¹⁰ stehen für H oder N(Alk¹)₂. Ganz besonders bevorzugt ist es, wenn zumindest einer der verbleibenden Substituenten R¹ bis R¹⁰ steht für N(Alk¹)₂ und die übrigen verbleibenen Substituenten stehen für H. Hierbei ist es bevorzugt, wenn genau ein verbleibender Substituent R¹ bis R¹⁰ für N(Alk¹)₂ steht und die übrigen verbleibenden Substituenten für H stehen.

In den vorhergehend beschriebenen Definitionen für die verbleibenden Substituenten R¹ bis R¹⁴ kann Alk¹ bevorzugt für Alk² und besonders bevorzugt für Alk³ stehen.

Bevorzugt stehen R¹¹ und R¹² jeweils unabhängig voneinander für OH oder für Alk³. Ganz besonders bevorzugt steht ein Substituent R¹¹ oder R¹² für OH und der andere Substituent für H.

Dabei versteht man unter einem verbleibenden Substituenten R¹ bis R¹⁴ diejenigen Substituenten R¹ bis R¹⁴, die nicht durch einen Substituenten der Formel IIIa, IIIb oder IIIc substituiert sind.

Bevorzugt sind Verbindungen, bei denen zumindest 2 der R¹ bis R⁵ stehen für H, besonders bevorzugt Verbindungen, bei denen zumindest 3 der R¹ bis R⁵ stehen für H und ganz besonders bevorzugt Verbindungen, bei denen zumindest 4 der R¹ bis R⁵ stehen für H, wobei die restlichen Substituenten R¹ bis R⁵ die Bedeutung der vorhergehend beschriebenen verbleibenden Substituenten R¹ bis R⁵ haben.

Bevorzugt sind Verbindungen, bei denen zumindest 2 Subsituenten der Substutenten R⁶ bis R¹⁰ für H stehen, besonders bevorzugt sind Verbindungen, bei denen zumindest 3 Substituenten der Substituenten R⁶ bis R¹⁰ für H stehen und ganz besonders bevorzugt sind Verbindungen, bei denen zumindest 4 Substituenten der Substituenten R⁶ bis R¹⁰ für H stehen, wobei die restlichen Substituenten R⁶ bis R¹⁰ die Bedeutung der vorhergehend beschriebenen verbleibenden Substituenten R⁶ bis R¹⁰ haben.

Dabei versteht man unter einem restlichen Substituenten R¹ bis R¹⁰ diejenigen Substituenten R¹ bis R¹⁰, die nicht durch einen Substituenten der Formel IIIa, IIIb oder IIIc substituiert sind oder die nicht H sind.

Bevorzugt sind Verbindungen mit einem Substituenten der Formel IIa für X oder mit einer Einfachbindung für X.

Besonders bevorzugt sind Verbindungen, bei denen genau einer der Substituenten R¹ bis R⁵ oder genau einer der Substituenten R⁶ bis R¹⁰ für einen Substituenten der Formel IIIa, IIIb oder IIIc steht.

Bevorzugt sind Verbindungen, bei denen genau einer der Substituenten R¹ bis R⁵ oder genau einer der Substituenten R⁶ bis R¹⁰ für einen Substituenten der Formel IIIa oder IIIb steht.

R¹⁵ steht für einen Ascorbinsäurerest der Formel IVa oder IVb oder einen Dehydroascorbinsäurerest der Formel IVc oder IVd, wobei die Ascorbinsäure in der D- oder L-Form oder als ein, insbesondere nicht äquimolares, Gemisch der beiden enantiomeren Formen vorliegen kann.

R¹⁵ steht bevorzugt für einen Ascorbinsäurerest der Formel IVa-1 oder IVb-1, wie zuvor beschrieben.

Besonders bevorzugt ist der 6-O-Ester der Ascorbinsäure und somit steht R¹⁵ besonders bevorzugt für einen Ascorbinsäurerest der Formel IVb, wobei die Ascorbinsäure in der D- oder L-Form oder als ein, insbesondere nicht äquimolares, Gemisch der beiden enantiomeren Formen vorliegen kann.

Ganz besonders bevorzugt steht R¹⁵ für einen L-Ascorbinsäurerest der Formel IVb-1.

Besonders bevorzugte Verbindungen der Formel I sind 4-Phenylazobenzoesäure-(R)-2-((R)-3,4-dihydroxy-5-oxo-2,5-dihydro-furan-2-yl)-2-hydroxy-ethylester, 4-(4-Dimethylamino-phenylazo)-benzoesäure-(R)-2-((R)-3,4-dihydroxy-5-oxo-2,5-dihydro-furan-2-yl)-2-hydroxy-ethylester, 4-(4-Dimethylamino-phenylazo)-phenylsulfonsäure-2-(R)-((R)-(3,4-dihydroxy-5-oxo-2,5-dihydro-furan-2-yl)-2-hydroxy-ethylester oder 2-(5-Hydroxy-3-methyl-1-phenyl-1H-pyrazol-4-ylazo)-benzoesäure(R)-2-((R)-3,4-dihydroxy-5-oxo-2,5-dihydro-furan-2-yl)-2-hydroxy-ethylester.

Eine ganz besonders bevorzugte Verbindung der Formel I ist 4-Phenylazobenzoesäure-(R)-2-((R)-3,4-dihydroxy-5-oxo-2,5-dihydrofuran-2-yl)-2-hydroxy-ethylester.

Verbindungen der Formel I weisen somit genau einen Ascorbinsäurerest der Formel IVa, IVb, IVa-1 oder IVb-1 oder einen Dehydroascorbinsäurerest der Formel IVc, IVd, IVc-1 oder IVd-1 als Linker auf und einen Rest einer Azoverbindung der Formel V, wie nachfolgend beschrieben. Der Linker Ascorbinsäure bzw. der Linker Dehydroascorbinsäure verbessert die Anbindungsfähigkeit der Verbindungen der Formel I, da auch mittels des jeweiligen Linkers die Verbindung der Formel I an die jeweilige Matrix assoziert und/oder eine kovalente Bindung zur jeweiligen Matrix ausbildet werden kann. Unter Anderem dadurch weisen die Verbindungen der Formel I eine im Vergleich zu nachfolgend beschriebenen Azoverbindungen der Formel V deutlich verbesserte Anbindungsfähigkeit auf, wodurch eine Färbung von Matrices mit Verbiondungen der Formel I sich durch eine ausgeprägte Dauerhaftigkeit der Färbung, insbesondere gegenüber dem Auswaschen, auszeichnet.

Weiterhin stellen diese Linker natürliche Linker dar, deren Verträglichkeit, insbesondere für den Menschen, vorteilhaft gegeben ist. Somit sind in der Regel die Verbindungen der Formel I ebenfalls in ihrer Verträglichkeit, insbesondere für den Menschen, verbessert.

Überaschenderweise kann mit Verbindungen der Formel I zudem eine intensivere Färbung erreicht werden. Unter anderem dadurch ist die Ergiebigkeit im Färbeprozess erhöht, da bei einer gleichen molaren Einsatzkonzentration eine intensivere Färbung erzielt werden kann als mit den nachfolgend beschriebenen Azoverbindungen der Formel V. Matrices, die mit Verbindungen der Formel I gefärbt werden, zeichnen sich demzufolge durch eine intensivere Färbung aus. Vorteilhaft lassen sich dadurch Kosten, sowohl während des Färbeprozesses als auch hinsichtlich einer notwendigen Wiederholung des Färbens, deutlich reduzieren.

Zudem sind die Verbindungen der Formel I farbstabil und fotostabil. Sie zeichnen sich außerdem durch verbesserte biologische Abbaubarkeiten aus und sind somit umweltverträglicher.

Beispielhaft zeigt eine Verbindung A mit zwei Ascorbinsäureresten ein geringeres Dipolmoment als eine Verbindung B mit nur einem Ascorbinsäurerest. Die Dipolmomente sind den Verbindung nachstehend in Klammern angegeben.

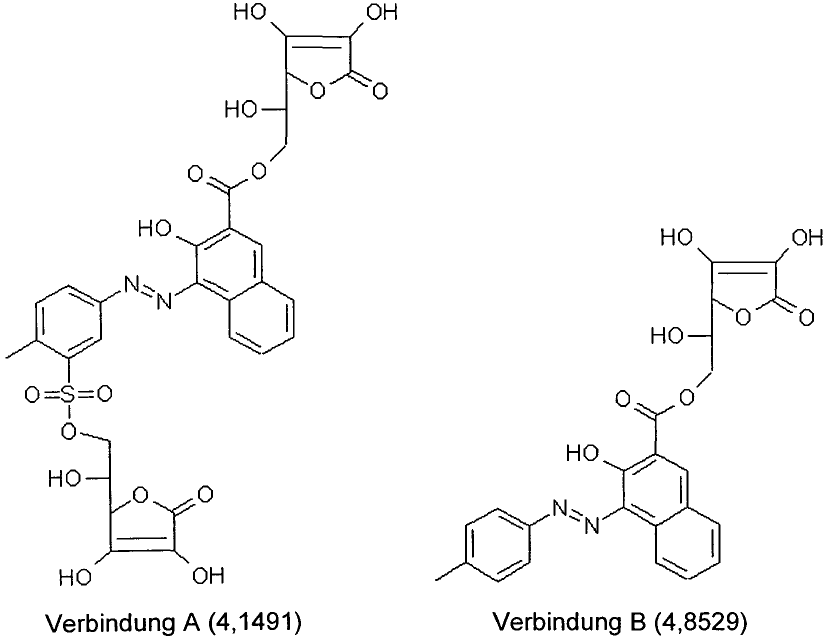

Dabei wurden die Dipolmomente der beiden Verbindungen A und B mittels der Software Gaussian 03W der Firma Gaussian Inc. Quantensimulationen ermittelt. Dazu wurde mittels der semiempirischen quantenchemischen Methode AM ("Austin Model 1") die Molekulargeometrie optimiert und dann die TD-DFT (Time dependent density function theorie) angewendet, wobei mittels der Dichtefunktionaltheorie-Methode B3PW91 und dem Funktionenbasisset 6-31G(d) die Kalkulationen für die Dipolmomente durchgeführt wurden (relative Einheiten). Für Verbindung B ermittelt man so ein mit 4,8529 deutlich höheres Dipolmoment als für Verbindung A, deren Dipolmoment bei 4,1491 liegt.

Somit sind die Verbindungen nach Formel I, wie zuvor beschrieben, gegenüber Verbindungen mit mehreren Ascorbinsäureresten vorteilhaft zumindest hinsichtlich ihres auftretenden höheren Dipolmomentes verbessert, so dass Verbindungen der Formel I gegenüber Verbindungen mit mehr als einem Ascorbinsäurerest eine verbesserte Amphiphilie aufweisen können.

Durch Einsatz genau eines Ascorbinsäurerestes der Formel IVa, IVb, IVa-1 oder IVb-1 oder eines Dehydroascorbinsäurerestes der Formel IVc, IVd, IVc-1 oder IVd-1 als Linker können somit Verbindungen der Formel I amphiphiler sein, weshalb die Penetration der Verbindungen der Formel I in die Matrix leichter stattfindet. Unter anderem dadurch kann eine vollständigere und länger anhaltendere Färbung erzielt werden, so dass auch aufgrund der Tiefenwirkung der Verbindungen der Formel I eine Färbung mit Verbindungen der Formel I, wie zuvor beschrieben oder als bevorzugt beschrieben, ergiebiger ist.

Zudem wird aufgrund der hohen Amphiphilie und des großen Wasserbindungsvermögens der Verbindungen der Formel I die Feuchtigkeit einer mit zumindest einer Verbindung der Formel I behandelten Matrix deutlich verbessert. Dies führt zu verbesserten Matrixstruktureigenschaften und zu einer erhöhten Elastizität der Matrix.
Bezogen auf die bevorzugte Matrix Haar gelten insbesondere folgende Aussagen: die Verbindungen der Formel I sowie die als bevorzugt angegebenen Verbindungen der Formel I, färben das Haar intensiver im Vergleich zu dem Farbstoff ohne Ascorbinsäurelinker. Die Verbindungen der Formel I sowie die als bevorzugt angegebenen Verbindungen der Formel I sind geeignet, um durch die Haarkutikula in den Haarkortex vorzudringen, wodurch eine vollständige und anhaltende Haarfärbung über das gesamte Haar erzielbar wird. Desweiteren verbessern die Verbindungen der Formel I sowie die als bevorzugt angegebenen Verbindungen der Formel I die Haarfeuchtigkeit durch hervorragende Hydratisierung des Haarkeratins. Mit der Haarfeuchtigkeit einhergehend verbessern sich somit die Haarsstruktureigenschaften, insbesondere die Haarelastizität.

Ein weiterer Gegenstand der Erfindung ist ein Verfahren zur Herstellung einer Verbindung der Formel I, wie zuvor beschrieben, bei dem in einem

Veresterungsschritt genau eine Säuregruppe der Formel Vlla, Vllb oder VIIc einer Azoverbindung der Formel V, 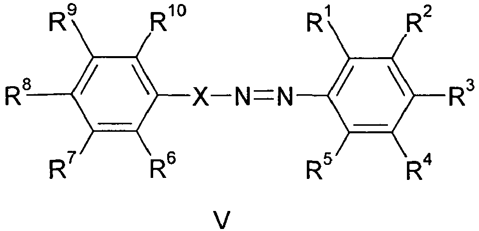 wobei X steht für eine Einfachbindung oder einen Substituenten der Formel VIa oder Vlb 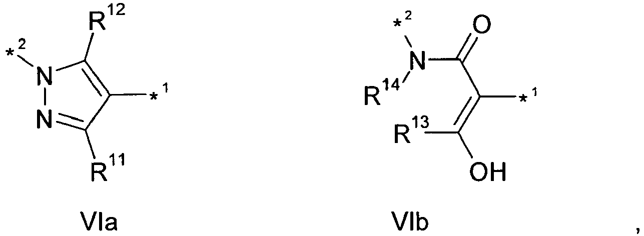 wobei *¹ zur N=N orientiert ist,
wobei genau einer der Substituenten R¹ bis R¹⁴ jeweils unabhängig voneinander steht für eine Säuregruppe der Formel Vlla, Vllb oder Vllc 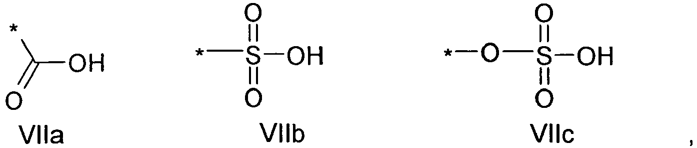 wobei die verbleibenden Substituenten R¹ bis R¹⁴ eine vorhergehend beschriebene Bedeutung haben, mit genau einer Hydroxygruppe auf der fünften oder sechsten Position der Ascorbinsäure oder der Dehydroascorbinsäure umgesetzt wird.

Somit wird der Linker Ascorbinsäure, bevorzugt L-Ascorbinsäure, oder Dehydroascorbinsäure, bevorzugt L-Dehydroascorbinsäure, über eine seiner OH-Gruppen der fünften und/oder sechsten Position mit der Azoverbindung der Formel V durch Veresterung verbunden. Erfindungsgemäß kommen deshalb Azoverbindungen der Formel V in Frage, die genau eine Carbonsäure-, eine Sulfonsäure- oder eine HO(SO₂)O-Gruppe aufweisen oder deren Salze oder aktivierte Formen, wie zum Beispiel Aktivester, Säurechloride oder dergleichen.

Vor dem Veresterungsschritt kann in einem Aktivierungsschritt die Säuregruppe der Formel Vlla, VIIb oder Vllc der jeweiligen Azoverbindung der Formel V für eine nachfolgende Veresterung aktiviert werden. Zu diesem Zweck kann aus der Azoverbindung der Formel V ein Aktivester, ein Säurechlorid oder dergleichen hergestellt werden.

Des Weiteren kann vor dem Veresterungsschritt und gegebenenfalls. vor dem Aktivierungsschritt zumindest eine Hydroxygruppe auf der fünften oder sechsten Position der Ascorbinsäure oder der Dehydroascorbinsäure mittels einer ersten Schutzgruppe geschützt werden. Ebenso kann des Weiteren zumindest eine Hydroxygruppe auf der zweiten oder dritten Position der Ascorbinsäure durch eine zweite Schutzgruppe geschützt werden.

Nach dem Veresterungsschritt kann zumindest eine erste und/oder eine zweite Schutzgruppe abgespalten werden, wobei die erste und die zweite Schutzgruppe unter unterschiedlichen Reaktionsbedingungen abspaltbar ist.

Als Schutzgruppen können die üblicherweise bekannten und oftmals verwendeten Schutzgruppen wie zum Beispiel, Methoxymethyl-, Benzyl-, SilylGruppen oder dergleichen zum Einsatz kommen.

Ein weiterer Kerngedanke der Erfindung ist die Verwendung von Verbindungen der Formel I, wie zuvor beschrieben, als Farbstoff für eine, insbesondere proteinhaltige, Matrix.

Dabei versteht man unter Matrix eine polymere Verbindung, die als funktionelle Gruppe zumindest eine freie NH-, NH₂-, SH- oder OH-Gruppen aufweist. Unter anderem aufgrund der funktionellen Gruppen der Matrix ist das Farbstoffderivat in der Lage, sich an die Matrix zu assoziieren und gegebenenfalls mit der Matrix kovalente Bindung auszubilden. Bevorzugte Matrices sind hierbei proteinhaltige Matrices. Besonders bevorzugt sind Haut, Haare und/oder Nägel und ganz besonders bevorzugt sind Haare. Ebenfalls geeignet sind Matrices wie zum Beispiel isolierte Proteine oder Gelatine. Des Weiteren sind ebenfalls geeignet synthetische polymere Verbindungen, die zumindest eine der oben aufgeführten funktionellen Gruppen aufweist. Demzufolge lassen sich die Verbindungen der Formel I auch zum Färben von Textilien, einschließlich Kunststofffasern, oder ganz allgemein von Kunststoffen verwenden. Hinsichtlich der Textilfärbung verwendbar sind insbesondere Fasern enthaltend Wolle, Baumwolle oder Seide.

Bevorzugt werden zumindest zwei Verbindungen der Formel I verwendet, wobei die Verbindungen sich hinsichtlich R¹⁵ unterscheiden und R¹⁵ steht für einen Ascorbinsäurerest der Formel IVa oder IVb oder für einen Dehydroascorbinsäurerest der Formel IVc oder IVd jeweilig als L- oder D-Enatiomer oder bevorzugt für einen Ascorbinsäurerest der Formel IVa-1 oder IVb-1 oder einen Dehydroascorbinsäurerest der Formel IVc-1 oder IVd-1.

Hergestellt werden können solche L/D-Gemische durch Veresterung einer Azoverbindung der Formel V mit einem racemischen, insbesondere nicht äquimolaren Gemisch von L- und D-Ascorbinsäure und/oder L-und D- Dehydroascorbinsäure, bevorzugt mit L-Ascorbinsäure.

Ein weiterer Erfindungsgedanke ist ein Verfahren zum Färben einer, insbesondere proteinhaltigen, Matrix, bei dem in einem Färbeschritt direkt durch Einwirken einer Dispersion und/oder Lösung und/oder Emulsion einer Verbindung der Formel I, wie zuvor beschrieben, auf die Matrix dieselbe gefärbt wird.

Dabei kann in einem Vorbehandlungsschritt die Matrix zur Beeinflussung und insbesondere zur Verbesserung des Färbeverhaltens derselben mittels eines Vorbehandlungsmittels vorbehandelt werden. Ein solches Vorbehandlungsmittel kann basisch, sauer oder neutral sein, eine oxidative Wirkung, zum Beispiel durch Vorhandensein eines Oxidationsmittels wie Wasserstoffperoxid, aufweisen und gegebenenfalls Wasser enthalten. Üblicherweise wird der Vorbehandlungsschritt vor dem Färbeschritt durchgeführt.

Gleichzeitig sind Verbindungen der Formel I wegen ihrer Ascorbinsäureabstammung sehr gute Antioxidantien. Dies ist von Vorteil, da Färbeprozeduren, insbesondere Haarfärbeprozesse, unter Bedingungen, z.B. alkalischen Bedingungen in Gegenwart von Oxidationsmitteln wie Wasserstoffperoxid und/oder Ammoniumperoxid, ablaufen, die zur Ausbildung reaktiver Sauerstoff- und/oder Stickstoff und/oder Kohlenstoffspecies führen (z.B. ROS reactive oxygen species). Diese können anhaftende Gewebe wie Wolle, Haut oder Haar schädigen. Antioxidantien wirken diesem Effekt entgegen.

Ein weiterer Gegenstand der Erfindung sind Zubereitungen, die zumindest eine Verbindung der Formel I, wie zuvor beschrieben, enthalten. Weiterhin können diese Zubereitungen zumindest einen für kosmetische, pharmazeutische, dermatologische Zubereitungen oder Haushaltsprodukte geeigneten Träger enthalten.

In bevorzugten Ausführungsformen wird die mindestens eine Verbindung der Formel I mit den definierten oder als bevorzugt angegebenen Substituenten in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt.

Ein weiterer Gegenstand der Erfindung ist ein Verfahren zur Herstellung einer solchen Zubereitung, wie zuvor beschrieben, wobei zumindest eine Verbindung der Formel I mit zumindest einem für kosmetische, pharmazeutische, dermatologische Zubereitungen oder Haushaltsprodukte geeigneten Träger und gegebenenfalls Hilfsstoffen und/oder Füllstoffen gemischt, insbesondere dispergiert und/oder emulgiert und/oder gelöst wird. Geeignete Trägerstoffe sowie Aktiv- oder Hilfsstoffe sind im nachfolgenden Teil ausführlich beschrieben.

Bei den Zubereitungen handelt es sich dabei üblicherweise um Zubereitungen zur Anwendung an Textilien oder zur Anwendung an Haut und Haar von Mensch und Tier. Beispielhaft sind kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Weitere bevorzugte Kombinationen von Ausführungsformen sind in den Ansprüchen offenbart.

In den beschriebenen Zubereitungen, die erfindungsgemäß mindestens eine Verbindung nach Formel I enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Bevorzugte Zubereitungen können neben den Verbindungen nach Formel I organische UV-Filter enthalten, sogenannte hydrophile oder lipophile Sonnenschutzfilter, die im UVA-Bereich und/oder UVB-Bereich und(/oder IR und/oder VIS-Bereich (Absorber) wirksam sind. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische wie auch anorganische UV Filter sind in den Patentanmeldungen EP-A 0 487 404 sowie WO2009/077356 angegeben.

Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzo-triazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. BASF.

Triazin Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine oder 2,4,6-Tris-(biphenyl)-1,3,5-triazine.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

### Piperazinderivate wie beispielsweise die Verbindung

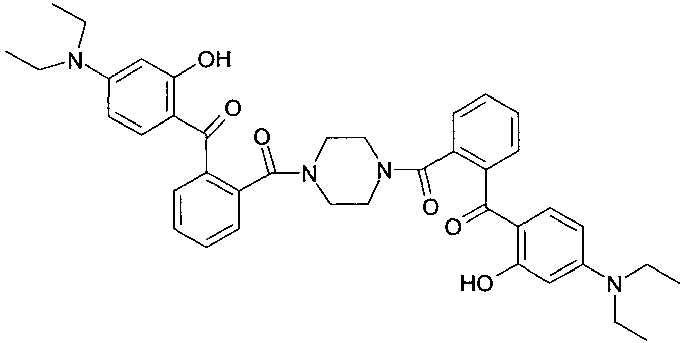

oder die UV-Filter der folgenden Strukturen:

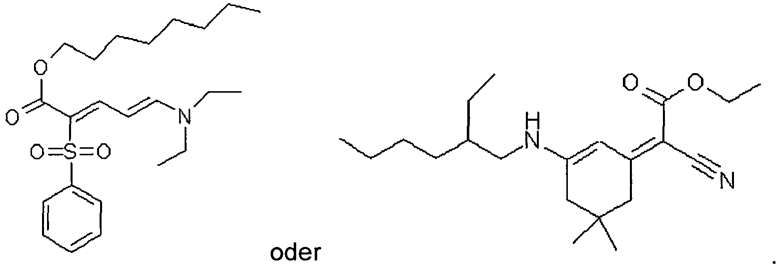

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

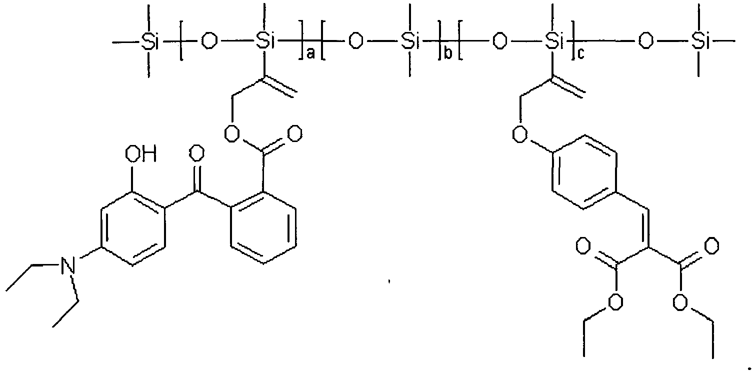

Die in der Liste aufgeführten Verbindungen sind nur als Beispiele aufzufassen. Selbstverständlich können auch andere UV-Filter verwendet werden.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylene-bis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Bevorzugte Zubereitungen können neben den Verbindungen der Formel I sowie den gegebenenfalls anderen organischen UV-Filtern, wie zuvor beschrieben, weitere anorganische UV-Filter enthalten, sogenannte partikuläre UV-Filter.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   ∘ "Zinc Oxide CS-5" der Fa. Toshibi (ZnO nachbehandelt mit polymethylhydrogenosiloxane)
   ∘ Nanogard Zinc Oxide FN der Fa. Nanophase Technologies
   ∘ "SPD-Z1" der Fa Shin-Etsu (ZnO nachbehandelt mit einem Silikongepfropften Acrylpolymer, dispergiert in Cyclodimethylsiloxane
   ∘ "Escalol Z100" der Fa ISP (Aluminiumoxid nachbehandeltes ZnO dispergiert in einer ethylhexyl methoxycinnamate/PVPhexadecene/methicone copolymer Mischung)
   o "Fuji ZNO-SMS-10" der Fa. Fuji Pigment (ZnO nachbehandelt mit Siliciumdioxid und Polymethylsilesquioxan);
   o Unbehandeltes Ceroxide Mikropigment z.B. mit der Bezeichnung "Colloidal Cerium Oxide" der Fa Rhone Poulenc
   o Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Ferner sind erfindungsgemäße Farbstoffe mit allen Wirkstoffen und Hilfstoffen kombinierbar, wie sie in WO2009/098139 systematisch gelistet sind. Insbesondere gehören diese Stoffe zu den darin aufgeführten Verwendungskategorien "Moisturizers and Humectants", "Desquamating agents", "Agents for improving the barrier function", "Depigmenting Agents", "Antioxidants", "Dermo-relaxing or dermo-decontracting agents", "Anti-glycation agents", "Agents for stimulating the synthesis of dermal and/or epidermal macromolecules and/or for preventing their degradation", "Agents for stimulating fibroblast or keratinocyte proliferation and/or keratinocyte differentiation", "Agents for promoting the maturation of the horny envelope", "NO-synthase inhibitors", "Peripheral benzodiazepine receptor (PBR) antagonists", "Agents for increasing the activity of the sebaceous glands", "Agents for stimulating the energy metabolism of cells", "Tensioning agents", "Fat-restructuring agents", "Sliming agents", "Agents for promoting the cutaneous microcirculation", "Calmatives or anti-irritants", "Sebo-regulating or anti-seborrhoic agents", "Astringents", "Cicatrizing agents", "Anti-inflammatory agents", "Antiacne agents".

Die schützende Wirkung von Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B 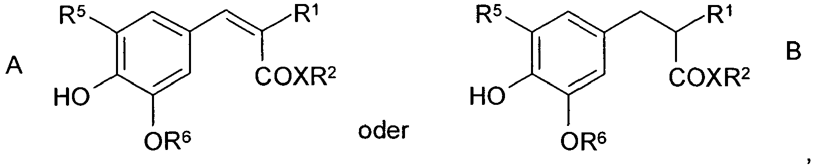 worin
R¹ aus der Gruppe -C(O)CH₃, -CO₂R³, -C(O)NH₂ und -C(O)N(R⁴)₂ ausgewählt werden kann,
X O oder NH,
R² lineares oder verzweigtes Alkyl mit 1 bis 30 C-Atomen,
R³ lineares oder verzweigtes Alkyl mit 1 bis 20 C-Atomen,
R⁴ jeweils unabhängig voneinander H oder lineares oder verzweigtes Alkyl mit 1 bis 8 C-Atomen,
R⁵ H oder lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäurebis-(2-ethylhexyl)ester (z.B. Oxynex^{®} ST Liquid) und/oder 2-(4-Hydroxy-3,5-dimethoxybenzyl)-malonsäure-bis-(2-ethylhexyl)ester (z.B. RonaCare^{®} AP).

Ferner ist die Kombination mit 2-(4-Hydroxy-3-methoxybenzyliden)-malonsäure-bis-isopropylester bzw. 2-(4-Hydroxy-3-methoxybenzyl)-malonsäure-bis-isopropylester (hydrogenated diisopropyl vanilidene malonate) bevorzugt. Analoges gilt für entsprechende Bis-ethylester.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf.
Häufig wird Quercetin (Cyanidanol, Cyanidenolon 1522, Meletin, Sophoretin, Ericin, 3,3',4',5,7-Pentahydroxyflavon) als besonders wirksames Antioxidans genannt.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als anti-aging Wirkstoffe Produkte der Firma Merck wie z.B. 5,7-Dihydroxy-2-methyl-chromon, vermarktet unter dem Handelsnamen RonaCare®Luremine oder die Handelsprodukte Ronacare®Isoquercetin, Ronacare©Tilirosid oder Ronacare®Cyclopeptide 5 verwendet werden.

Ferner können die erfindungsgemäßen Zubereitungen mindestens einen Selbstbräuner als weiteren Inhaltsstoff enthalten.
Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin oder Rucinol.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methylcyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzwegten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die erfindungsgemäße Mischung kann in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasserin-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol-(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Beispielen dargestellt und werden in der nachfolgenden Beschreibung näher erläutert, ohne den Umfang der vorliegenden Erfindung zu beschränken.

### Zubereitungen:

### Allgemeine Hinweise:

Beschriebene Ascorbate der Formel I von Azoverbindungen der Formel V können sowohl in die Ölphase von Zubereitungen, wie z.B. Emulsionen eingearbeitet werden als auch in deren Wasserphase. Bevorzugt ist auch eine kombinierte Einarbeitung in Öl- und Wasserphase möglich. Zur Einarbeitung in Öl- oder Wasserphase ist die Verwendung von Löslichkeitsvermittlern von Vorteil. Dabei ist z.B. der Zusatz von Alkoholanteilen vorteilhaft (e.g. Ethanol, Isopropanol). Der pH-Wert der Formulierung sollte bevorzugt zwischen pH=3 und pH=7 liegen, um durch saure Umgebung in der Formulierung eine zufriedenstellende Stabilität des Ascorbats zu erzielen. Besonders bevorzugt ist es z.B. den pH-Wert der Wasserphase mit Citratpuffer auf pH=5 zu puffern, da dies dem natürlichen pH-Wert der Haut entspricht. Generell können die beschriebenen Ascorbate derart in mindestens eine lipophile oder hydrophile Phase einer Zubereitung eingearbeitet werden, dass entweder eine klare Lösung vorliegt oder die Substanzen dispergiert vorliegen.

In den nachfolgenden Rezepturbeispielen werden folgende Substanzcodes verwendet:
Farbstoff A: 4-Phenylazobenzoesäure-(R)-2-((R)-3,4-dihydroxy-5-oxo-2,5-dihydro-furan-2-yl)-2-hydroxy-ethylester 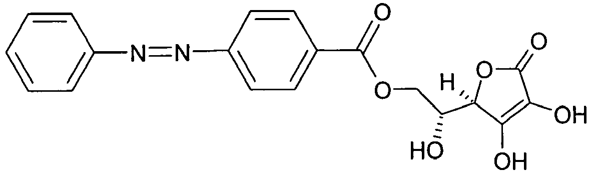
Farbstoff B: 4-(4-Dimethylamino-phenylazo)-benzoesäure-(R)-2-((R)-3,4-dihydroxy-5-oxo-2,5-dihydro-furan-2-yl)-2-hydroxy-ethylester 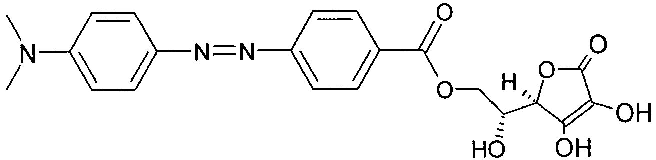
Farbstoff C: 4-(4-Dimethylamino-phenylazo)-phenylsulfonsäure-2-(R)-((R)-(3,4-dihydroxy-5-oxo-2,5-dihydro-furan-2-yl)-2-hydroxy-ethylester 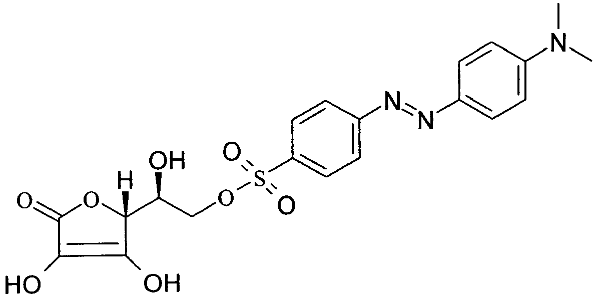
Farbstoff D: 2-(5-Hydroxy-3-methyl-1-phenyl-1H-pyrazol-4-ylazo)-benzoesäure(R)-2-((R)-3,4-dihydroxy-5-oxo-2,5-dihydro-furan-2-yl)-2-hydroxy-ethylester

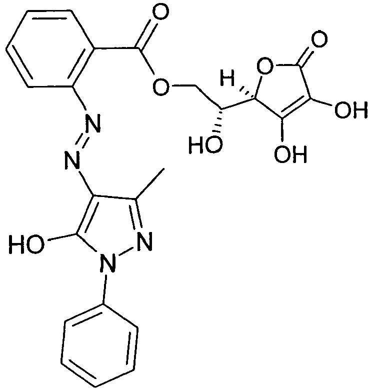

### Synthesebeispiele:

### Synthesebeispiel 1: Synthese Farbstoff A

In einem Gemisch aus 12 ml NMP (N-Methyl-2-pyrrolidon) und 26 ml Acetonitril werden 2,5 g para-Phenylazobenzoesäure (11,05 mmol, 1 Äq.) gelöst. Es werden bei Raumtemperatur 0,96 ml Thionylchlorid (13,26 mmol, 1,2 Äq.) zugegeben und für 3 h bei Raumtemperatur gerührt. Anschließend werden 3,9 g L-Ascorbinsäure (22 mmol, 2 Äq.) zugegeben und für 16 Stunden bei Raumtemperatur gerührt. Zur Reaktionslösung werden 70 ml 2N HCl gegeben. Der ausgefallene Feststoff wird abgesaugt und mit 50 ml Dichlormethan suspendiert. Nach Filtration und Trocknung erhält man so mit 66% Ausbeute (2,7 g) das Produkt als orangebraunen Feststoff.

¹H-NMR (400 MHz, DMSO) δ = 4.19 (t, CH, *J* = 5.5 Hz), 4.32 (dd, CH₂, *J* = 5.5 Hz, *J* = 10.5 Hz), 4.41 (dd, CH₂, *J* = 5.5 Hz, *J* = 10.5 Hz), 4.79 (d, CH, *J* = 1.2 Hz), 5.52 (br, OH), 7.47 (m, 2 x Ar-H), 7.82 (m, 2 x Ar-H), 7.87 (d, 2 x Ar-H, *J* = 8.3 Hz), 8.13 (d, 2 x Ar-H, *J* = 8.3 Hz), 11.15 (br, OH) ppm.

¹³C-NMR (75 MHz, DMSO) δ = 65.68, 65.80, 75.14, 118.27, 122.58, 122.83, 129.55, 130.59, 131.52, 132.26, 151.89, 152.01, 154.50, 164.93, 170.27 ppm.

### Synthesebeispiel 2: Synthese Farbstoff B:

In einem Gemisch aus 15 ml NMP und 30 ml Acetonitril werden 2,5 g para- 4-(4-Dimethylamino-phenylazo)-benzoesäure 9,28 mmol, 1 Äq.) suspendiert. Es werden bei Raumtemperatur 0,808 ml Thionylchlorid (11,14 mmol, 1,2 Äq.) zugegeben und für 3 h bei Raumtemperatur gerührt. Anschließend werden 3,27 g L-Ascorbinsäure (19 mmol, 2 Äq.) zugegeben und für 24 Stunden bei Raumtemperatur gerührt. Zur Reaktionslösung werden 70 ml 2N HCl gegeben. Der ausgefallene Feststoff wird abgesaugt und mit 80 ml Dichlormethan suspendiert. Nach Filtration und Trocknung erhält man so mit 76% Ausbeute (3 g) das Produkt als rotbraunen Feststoff.

### Synthesebeispiel 3: Synthese Farbstoff C:

5,6-Isopropyliden-L-ascorbat (10 g; 46,3 mmol, 1 Äq.) wird in 30 ml THF und 35 ml DMSO gelöst, 19,2 g Kaliumcarbonat zugegeben und 13 ml Benzylbromid (110 mmol, 2,4 Äq.) zugetropft. Nach 3 Std. bei 50°C ist die Gasentwicklung abgeschlossen. Der Feststoff wird abfiltriert und 3 Mal mit je 100 ml Essigester extrahiert. Die vereinigten organischen Phasen werden über Natriumsulfat getrocknet und das Lösungsmittel im Vakuum entfernt. Man erhält 2,3-Dibenzyl-5,6-Isopropyliden-ascorbat ohne weitere Aufreinigung nahezu quantitativ.
Das zweifach Benzyl-geschützte 5,6-Isopropyliden-L-ascorbat wird in 65 ml THF gelöst und 30 ml 2 N HCl werden langsam bei Raumtemperatur zugegeben. Nach 48 Std. wird mit 150 ml MTBE (Methyl-tert.-butylether) und festem Natriumchlorid bis zur Sättigung versetzt und extrahiert. Die organische Phase wird über Natriumsulfat getrocknet und das Lösungsmittel im Vakuum entfernt. Man erhält 2,3-Dibenzyl-L-ascorbat ohne weitere Aufreinigung nahezu quantitativ.
Die zweifach Benzyl-geschützte L-Ascorbinsäure (2,14 g; 6 mmol, 1 Äq.) wird in 11 ml Dichlormethan gelöst. Dann werden bei 0°C Triethylamin (0,91 g; 9 mmol, 1,5 Äq.) und Dabsylchlorid (2,3 g; 7,2 mmol, 1,2 Äq.) zugeben. Es wird auf Raumtemperatur (RT) erwärmt und nach 22 Std. das Lösungsmittel im Vakuum entfernt. Der Rückstand wird mit 50 ml Essigsäureethylester und 50 ml ges. NaCl-Lsg. extrahiert Die organische Phase wird über Natriumsulfat getrocknet und das Lösungsmittel im Vakuum entfernt. Das entstandene Rohprodukt wird in 50 ml Essigester gelöst und unter 5 bar Wasserstoffdruck mit 2 Gew% Pd-C-Katalysator reduziert. Nach Filtration des Katalysators wird das Produkt durch Filtration über 300 g Kieselgel und 1500 ml Ethylacetat gereinigt. Man erhält so den Farbstoff C als orangefarbener Feststoff.

### Synthesebeispiel 4: Synthese des Farbstoffs D:

Es werden 10 g 2-(5-Hydroxy-3-methyl-1-phenyl-1H-pyrazol-4-ylazo)-benzoesäure (31,03 mmol) in 120 ml Dioxan gelöst, 6,7 g DCC (Dicyclohexylcarbodiimid) (32,58 mmol, 1,05 Äq.) und anschließend portionsweise insgesamt 10,93 g L-Ascorbinsäure (62 mmol, 2 Äq.) zugegeben. Nach 13 h Reaktionszeit bei Raumtemperatur wird das Lösungsmittel zu 80% abdestilliert. Es werden 150 ml Wasser zugegeben und der entstandene Feststoff abfiltriert und mit Wasser gewaschen. Anschließend wird durch dreimaliges Suspendieren in je 100 ml iso-Propanol aufgereinigt. Man erhält als gelben Feststoff 8,3 g des Farbstoffs D (56%).

### Beispiel 1: Textilfärbung

Farbstoff B und Farbstoff A werden zu je 5% in Wasser gelöst/dispergiert. Der pH-Wert des Wassers ist alkalisch. Die wässrige Lösung/Dispersion wird zunächst aufgekocht und anchließend auf unter 40°C langsam abgekühlt, bevor man die Wolle hinzugibt (ca. 1 kg Wolle auf 25-30 Liter Wasser). Nach 24h wird die wolle herausgenommen und mit Leitungswasser gut gespült.

### Beispiel 2: Haarfarbe aus verschiedenen Komponenten:

### Konponente A:

Tocopherol, Linalool, Geraniol, Disodium EDTA, Parfum, ascorbic acid, alcohol denat., Sodium sulfite, Sodium hydroxide, Sodium cocoyl isethionate, Bis-ethylhexyl Hydroxydimethoxy Benzylmalonate, Sodium lauryl sulfate, Ammonia, Lanolin alcohol, Glycol distearate, Sodium laureth sulfate, Glyceryl stearate, Ceteary alcohol, Aqua.

### Komponente B:

Aqua, hydrogen peroxide, cetearyl alcohol, PPG-38-buteth-37, petrolatum, laureth-2, sodium cetearyl sulfate, salicylic acid, disodium phosphate, phosphoric acid, etidronic acid.

### Komponente C:

Ethanolische Lösung von Farbstoff A und/oder B (je 2 Gew.-%) zudem enthaltend Bis-ethylhexyl Hydroxydimethoxy Benzylmalonate (1 Gew.-%).

### Komponente D:

Ethanolische Lösung von Bis-ethylhexyl Hydroxydimethoxy Benzylmalonate (1 Gew.-%).

### Anwendung:

Zur Haarfärbung wird bevorzugt in folgender Reihenfolge vorgegangen: Zunächst wird das Haar mit Komponente C vorbehandelt, Anschließend werden die Komponenten B und C gemischt und auf das Haar appliziert. Nach erfolgter Färbung wird Komponente D appliziert.

### Beispiel 3: Haarfarbe aus verschiedenen Komponenten:

### Konponente A:

Tocopherol, Linalool, Geraniol, Disodium EDTA, Parfum, Toluene-2,5-diamine sulfate, ascorbic acid, alcohol denat., Sodium sulfite, Sodium hydroxide, Sodium cocoyl isethionate, Bis-ethylhexyl Hydroxydimethoxy Benzylmalonate, 2-Methylresorcinol, 6-Amino-m-cresol, 4-Amino-2-hydroxytoluol, 4-Amino-m-cresol, Sodium lauryl sulfate, Ammonia, Lanolin alcohol, Glycol distearate, Sodium laureth sulfate, Glyceryl stearate, Ceteary alcohol, Aqua.

### Komponente B:

Aqua, hydrogen peroxide, cetearyl alcohol, PPG-38-buteth-37, petrolatum, laureth-2, sodium cetearyl sulfate, salicylic acid, disodium phosphate, phosphoric acid, etidronic acid.

### Komponente C:

Ethanolische Lösung von Farbstoff A und /oder B (je 2 Gew.-%) zudem enthaltend Bis-ethylhexyl Hydroxydimethoxy Benzylmalonate (1 Gew.-%).

### Komponente D:

Ethanolische Lösung von Bis-ethylhexyl Hydroxydimethoxy Benzylmalonate (1 Gew.-%).

### Anwendung:

Zur Haarfärbung wird bevorzugt in folgender Reihenfolge vorgegangen: Zunächst wird das Haar mit Komponente C vorbehandelt, Anschließend werden die Komponenten B und C gemischt und auf das Haar appliziert. Nach erfolgter Färbung wird Komponente D appliziert.

Für alle Beispielrezepturen der Zubereitungen gilt, dass diese optional auch frei von UV-Filtern hergestellt werden können.

### Beispiel 4: W/O Emulsion

| | a | b | c | d | e |
|---|---|---|---|---|---|
| Cetyl PEG/PPG-10/1 dimethicone | 3,00 | 3,00 | 3,00 | 3,00 | 3,00 |
| (Abil EM 90) | | | | | |
| Polyglyceryl-4 isostearate | 1,50 | 1,50 | 1,50 | 1,50 | 1,50 |
| (Isolan GI 34) | | | | | |
| Butylphthalimide isopropylphthalimide | 5,00 | 5,00 | 5,00 | 5,00 | 5,00 |
| (Pelemol^{®} BIP) | | | | | |
| Dimethyl isosorbide | 5,00 | 5,00 | 5,00 | 5,00 | 5,00 |
| (Arlasolve DMI) | | | | | |
| Farbstoff A | 2,00 | | | | 1,00 |
| Farbstoff B | | 2,00 | | | 1,00 |
| Farbstoff C | | | 2,00 | | 1,00 |
| Farbstoff D | | | | 2,00 | 1,00 |
| Uvinul^{®} A Plus (DHHB) | | 1,00 | 1,00 | 1,00 | |
| Ascorbinsäure | | | 0,37 | 1,00 | 3,00 |
| Mineral Oil | 8,00 | 8,00 | 8,00 | 8,00 | 8,00 |
| Ethylhexyl stearate | 5,00 | 5,00 | 5,00 | 5,00 | 5,00 |
| (Tegosoft^{®} OS) | | | | | |
| Cyclomethicone (and) Aluminium/Magnesium Hydroxide Stearate | 5,00 | 5,00 | 5,00 | 5,00 | 5,00 |
| (Gilugel SIL 5) | | | | | |
| Preservative | 1,00 | 1,00 | 1,00 | 1,00 | 1,00 |
| Water | Ad 100 | Ad 100 | Ad 100 | Ad 100 | Ad 100 |
| NaCl | 0,50 | 0,50 | 0,50 | 0,50 | 0,50 |
| EDTA | 0,10 | 0,10 | 0,10 | 0,10 | 0,10 |
| Citronensäure q.S. | | | | | |

Herstellung: Pelemol^{®} BIP, Arlasolv DMI und Emulgatoren werden vorgelegt. Die farbstoffe A-D und Uvinul^{®} A Plus werden darin gelöst. Die restlichen Bestandteile der Ölphase werden zugegeben und homogen vermischt. Unter Rühren wird die mit Citronensäure auf pH=4-5 eingestellte Wasserphase einemulgiert. Anschließend wird homogenisiert. Die Emulsionen können unter schonenden Bedingungen bei Raumtemperatur hergestellt werden. Durch Erhöhung des Gehaltes an Ascorbinsäure können enthaltene Farbstoffascorbate stabilisiert werden. Idealerweise wird die Herstellung unter Inertisierung (Ausschluß von Sauerstoff hergestellt).

### Beispiel 5: Wasserfestes Sonnenschutzspray mit Auftragskontrolle

| A | | | |
|---|---|---|---|
| Farbstoff A | 1,00 | 1,00 | 2,00 |
| Diethylhexyl Syringylidenemabnate, Caprylic/Capric Triglyceride | | 0,50 | |
| (Oxynex^{®} ST Liquid) | | | |
| RonaCare^{®} AP | | 2,00 | |
| Ascorbyl Palmitate | | | 1,00 |
| Cyprylic/capric Triglyceride | 7,00 | 7,00 | 7,00 |
| (Miglyol 812 N) | | | |
| Butylphthalimide isopropylphthalimide | 9,00 | 9,00 | 9,00 |
| (Pelemol^{®} BIP) | | | |
| C12-15 alkyl benzoate (Tegosoft^{®} TN) | 10,00 | 10,00 | 10,00 |
| Phenethyl benzoate | 5,00 | 5,00 | 5,00 |
| (X-Tend 226) | | | |
| RonaCare^{®} Tocopherolacetat | 1,00 | 1,00 | 1,00 |

| B | | | |
|---|---|---|---|
| Cyclopentasiloxane | 43,80 | 41,30 | 41,80 |
| (Dow Corning 245) | | | |
| Phenyltrimethicone | 2,00 | 2,00 | 2,00 |
| (Dow Corning 556) | | | |
| Cyclopentasiloxane, dimethiconol | 20,00 | 20,00 | 20,00 |
| Dow Corning 1501 Fluid | | | |
| Parfümöl (q.s.) | 0,20 | 0,20 | 0,20 |

Herstellung: Die Komponenten der Phase A werden bei Raumtemperatur zusammengefügt und gerührt bis eine klare Lösung oder homogene Disperison vorliegt. Anschließend wird Phase B gemischt und unter Rühren zu Phase B gegeben. Man rührt weiter, bis letztlich das homogene Produkt vorliegt. Zu Zugabe von Antioxidantien wie Oxynex^{®} ST Liquid, RonaCare^{®} AP oder Ascorbylpalmitat kann die Stabilität der erfindungsgemäßen Substanzen erhöht werden.

### Beispiel 6: Pump Haartönungsspray

| A | | | |
|---|---|---|---|
| Farbstoff A | 1,00 | 1,00 | 4,00 |
| Farbstoff B | 1,00 | | |
| Farbstoff C | | 1,00 | |
| Farbstoff D | | | 1,00 |
| Ethanol 96% reinst | Ad 100 | Ad 100 | Ad 100 |
| PVPNA copolymer | 6,00 | 6,00 | 6,00 |
| PVPNA W 735 | | | |

| B | | | |
|---|---|---|---|
| Diethylhexyl Syringylidenemalonate, Caprylic/Capric Triglyceride | 0,06 | 0,25 | 0,50 |
| (Oxynex^{®} ST Liquid) | | | |
| PEG-75 Lanolin BHT | 0,20 | 0,20 | 0,20 |
| (Solan E - Low Dioxane) | | | |
| Parfum | 0,10 | 0,10 | 0,10 |
| (Frag 280853 Green Activating) | | | |

| C | | | |
|---|---|---|---|
| Wasser, demineralisiert | 13,00 | 13,00 | 13,00 |
| Titriplex III | 0,10 | 0,10 | 0,10 |
| PEG-12 dimethicone | 0,50 | 0,50 | 0,50 |
| Dow Corning 193 Fluid | | | |
| 0,1% D&C Red No 33 (CI 17200) in Wasser | 0,20 | 0,20 | 0,20 |
| PEG-40 Hydrogenated Castor Oil | 1,00 | 1,00 | 1,00 |
| (Cremophor RH 410) | | | |

Herstellung: Phase A vorlösen, bis eine klare Lösung vorliegt. Unter Rühren Phase B zu Phase A geben. Phase C vormischen und zum Rest geben, rühren, bis eine homogene Mischung entstanden ist.

### Beispiel 7: W/O-Emulsionen

| **Emulsion** | **A** | **B** | **C** | **D** | **E** | **F** |
|---|---|---|---|---|---|---|
| Polyglyceryl-2-Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |
| Bis-Ethylhexyloxyphenol Methoxyphenyltriazin | | 3 | 3 | 2 | | |
| Ethylhexyltriazon | | 4,5 | 3 | | 3 | |
| Farbstoff A | 1,0 | | 1,5 | 1,0 | 3,0 | |
| Diethylhexylbutamidotriazon | | | 1,5 | 4 | | |
| Butyl Methoxydibenzoylmethan | 2 | 3 | 4 | | 1 | 3 |
| Uvinul^{®} A Plus | | | | 4 | 2 | |
| Ethylhexylmethoxycinnamat | | | | | 7 | 5 |
| Farbstoff B | | 4,0 | 0,5 | 1,5 | | 0,5 |
| Taurin | 0,1 | | | 0,5 | 0,2 | |
| Vitamin E Acetat | 0,2 | 02 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Parfüm, Konservierungsmittel | q.s. | q.s | q.s | q.s | qs. | qs. |
| Farbstoffe, usw. | q.s. | q.s. | q.s. | q.s | q.s. | q.s. |
| Natriumhydroxid | q.s. | q.s. | q.s. | q.s | q.s. | q.s. |
| Wasser | ad 100,0 | ad 100,0 | ad 100,0 | ad 100,0 | ad 100,0 | ad 100,0 |

### Beispiel 8: Haarpflegeformulierung

| **Gehalt in g Komponente per 100 g Formulierung** | | | | | | |
|---|---|---|---|---|---|---|
| **Komponente** | **A** | **B** | **C** | **D** | **E** | **F** |
| Disodium EDTA | 0.100 | 0.100 | 0.100 | 0.100 | 0.100 | 0.100 |
| Oxynex^{®}ST | 2.000 | 2.000 | 2.000 | 2.000 | 2.000 | 2.000 |
| Farbstoff A | 0,10 | 0,25 | 0,50 | 1,50 | 2,00 | 4,00 |
| Farbstoff C | 0,50 | 1,00 | 1,50 | 2,00 | 2,50 | 3,00 |
| Hexamidine diisethionate | 0.100 | 0 | 0 | 0 | 0 | 0 |
| Tetrahydrocurcumin | 0 | 0.500 | 0 | 0 | 0 | 0 |
| Glycyrrhetinic acid | 0 | 0 | 0.300 | 0 | 0 | 0 |
| Thiotaine®¹ | 0 | 0 | 0 | 5.000 | 0 | 0 |
| N-undecylenoyl-L-phenylalanine | 0 | 0 | 0 | 0 | 1.000 | 0 |
| N-acetyl glucosamine | 0 | 0 | 0 | 0 | 0 | 2.000 |
| Niacinamide | 5.000 | 5.000 | 5.000 | 5.000 | 5.000 | 5.000 |
| Citric acid | 0.015 | 0 | 0 | 0 | 0 | 0 |
| Isohexadecane | 3.000 | 3.000 | 3.000 | 3.000 | 3.000 | 3.000 |
| Isopropyl isostearate | 1.330 | 1.330 | 1.330 | 1.330 | 1.330 | 1.330 |
| Isopropyl N-laurosylsarcosinate | 0 | 0 | 5.000 | 0 | 0 | 0 |
| Sucrose polycottonseedate | 0.670 | 0.670 | 0.670 | 0.670 | 0.670 | 0.670 |
| Polymethylsilsesquioxane | 0.250 | 0.250 | 0.250 | 0.250 | 0.250 | 0.250 |
| Cetearyl glucoside + cetearyl alcohol | 0.200 | 0.200 | 0.200 | 0.200 | 0.200 | 0.200 |
| Behenyl alcohol | 0.400 | 0.400 | 0.400 | 0.400 | 0.400 | 0.400 |
| Ethylparaben | 0.200 | 0.200 | 0.200 | 0.200 | 0.200 | 0.200 |
| Propylparaben | 0.100 | 0.100 | 0.100 | 0.100 | 0.100 | 0.100 |
| Cetyl alcohol | 0.320 | 0.320 | 0.320 | 0.320 | 0.320 | 0.320 |
| Stearyl alcohol | 0.480 | 0.480 | 0.480 | 0.480 | 0.480 | 0.480 |
| Tocopheryl acetate | 0.500 | 0.500 | 0.500 | 0.500 | 0.500 | 0.500 |
| PEG-100 stearate | 0.100 | 0.100 | 0.100 | 0.100 | 0.100 | 0.100 |
| Glycerin | 7.000 | 7.000 | 7.000 | 7.000 | 7.000 | 7.000 |
| Titanium dioxide | 0.604 | 0.604 | 0.604 | 0.604 | 0.604 | 0.604 |
| Polyacrylamide + C13-14 isoparaffin + laureth-7 | 3.000 | 2.000 | 2.000 | 2.000 | 2.000 | 2.000 |
| Panthenol | 1.000 | 1.000 | 1.000 | 1.000 | 1.000 | 1.000 |
| Benzyl alcohol | 0.400 | 0.400 | 0.400 | 0.400 | 0.400 | 0.400 |
| Dimethicone + dimethiconol | 2.000 | 2.000 | 2.000 | 2.000 | 2.000 | 2.000 |
| Water (to 100 g) | to 100 | to 100 | to 100 | to 100 | to 100 | to 100 |
| TOTAL | 100 | 100 | 100 | 100 | 100 | 100 |

### Beispiel 9: Haarpflegeformulierung

| **Gehalt in g Komponente per 100 g Formulierung** | | | |
|---|---|---|---|
| **Komponente** | **G** | **H** | **I** |
| Disodium EDTA | 0.100 | 0.100 | 0.100 |
| Oxynex^{®} ST | 2.000 | 2.000 | 2.000 |
| Farbstoff A, B, C, oder D oder farbstoffkombination aus A, B, C, oder D | 0.20 | 1.500 | 0,75 |
| Cetyl pyridinium chloride | 0.200 | 0 | 0 |
| Pitera^{®} | 0 | 10 | 0 |
| Ascorbyl glycoside | 0 | 0 | 2.000 |
| Niacinamide | 3.500 | 5.000 | 4.000 |
| Polyquaternium 37 | 0 | 0 | 0 |
| Isohexadecane | 3.000 | 2.500 | 2.000 |
| Isopropyl isostearate | 1.330 | 1.330 | 1.330 |
| Sucrose polycottonseedate | 0.670 | 0.670 | 0.670 |
| Polymethylsilsesquioxane | 0.250 | 0.250 | 0.250 |
| Cetearyl glucoside + cetearyl alcohol | 0.200 | 0.200 | 0.200 |
| Behenyl alcohol | 0.400 | 0.400 | 0.400 |
| Ethylparaben | 0.200 | 0.200 | 0.200 |
| Propylparaben | 0.100 | 0.100 | 0.100 |
| Cetyl alcohol | 0.320 | 0.320 | 0.320 |
| Stearyl alcohol | 0.480 | 0.480 | 0.480 |
| Tocopheryl acetate | 0.500 | 0.500 | 0.500 |
| PEG-100 stearate | 0.100 | 0.100 | 0.100 |
| Glycerin | 7.000 | 7.000 | 7.000 |
| Titanium dioxide | 0.604 | 0.604 | 0.604 |
| Polyacrylamide + C13-14 isoparaffin + laureth-7 | 2.000 | 2.000 | 2.000 |
| Panthenol | 1.000 | 1.000 | 1.000 |
| Benzyl alcohol | 0.400 | 0.400 | 0.400 |
| Dimethicone + dimethiconol | 2.000 | 2.000 | 2.000 |
| Water (to 100 g) | to 100 | to 100 | to 100 |
| TOTAL | 100 | 100 | 100 |

### Beispiel 10: O/W-Emulsionen

| **Emulsion** | **A** | **B** | **C** | **D** | **E** | **F** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 Dipolyhydroxystearat | | 0,8 | | | | 0,5 |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylengykol | | | 1 | | 5 | 3 |
| Glycerin | 3 | 5 | 7 | 10 | 3 | 3 |
| C18-C38 Säuretriglyceride | 0,5 | | 1 | | 1 | |
| Titandioxid | 5 | | | 2 | | |
| 2,2'-Methylen-bis-(6-(2H-benzotriazol-2-yl)-(1,1,3,3-tetramethylbutyl)phenol) | 2,5 | | | | | |
| 2,4,6-Tris-(biphenyl)-1,3,5-triazin | | 2 | | | | |
| Merocyanin an Gelatine gekoppelt | 6 | | 6 | | 10 | 3 |
| Benzotriazol an Gelatine gekoppelt | | 5 | | 10 | | 3 |
| C8-C16 Alkylpolyglycosid | 1 | 0,6 | | | | |
| UVASorb^{®} K2A | | | 2 | | | |
| Uvinul^{®} A Plus | 2 | | | | | 1 |
| Homosalat | | 5 | | 1 | | |
| Phenylbenzimidazol Sulfonsäure | | | 2 | | | 1 |
| Benzophenon-3 | *0,5* | | | | 1 | |
| Octylsalicylat | 5 | 5 | | 2 | | |
| Octocrylen | 2 | | | | 3 | 1 |
| Farbstoff A | 0,1 | 0,2 | 0,3 | 0,4 | 0,5 | 1,0 |
| Farbstoff B | 0,1 | 0,2 | 0,3 | 0,4 | 0,5 | 1,0 |
| Bis-Ethylhexyloxyphenol Methoxyphenyltriazin | | 3 | 2 | 1 | | |
| Parsol^{®} SLX | | | 3 | | | |
| Dihydroxyacetat | | | | | 4 | |
| Taurin | 0,1 | | | 0,5 | 0,2 | |
| 8-Hexadecen-1,16-dicarbonsäure | | 0,2 | | | | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel 11: O/W-Emulsionen

| **Emulsion** | **G** | **H** | **I** | **K** | **L** | **M** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F^{®} | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C₁₂₋₁₅ Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Diethylhexylnaphthalat | 2 | | | | | |
| Dicaprylcaprat | | 2 | | | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid | 5 | | 3 | 2 | | |
| Phenylbenzimidazol Sulfonsäure | 1 | | | 1 | 2 | 1 |
| Parsol® SLX | 0,5 | 1,0 | 1,5 | 2,0 | 2,5 | 3,0 |
| Farbstoff A | 0,5 | 1,0 | 3,0 | 0,5 | | |
| Farbstoff B | | | 0,5 | 3,0 | 1,0 | 0,5 |
| Kreatinin | 0,1 | 0,01 | 0,05 | | | |
| Kreatin | 0,5 | 0,2 | 0,1 | | | |
| Licorice Extrakt/Licochalkon | | | | 0,5 | | |
| Vitamin E Acetat | 0,2 | | | 0,5 | 0,5 | 0,5 |
| Tapioka Stärke | | 3 | | | 2 | |
| Na₂H₂EDTA | 0,1 | | 0,2 | | | 0,5 |
| Parfüm, Konservierungsmittel | q.s. | q.s. | q.s. | q.s. | q.s. | q.s. |
| Pigmentfarbstoffe | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel 12: O/W-Emulsionen zur Hautfärbung mit UV-Schutz

| **Emulsion** | **N** | **O** | **P** | **Q** | **R** | **S** |
|---|---|---|---|---|---|---|
| Glycerylstearat SE | | 2 | | 2 | | |
| Glycerylstearat | 2 | | 2 | | | |
| PEG-40 Stearat | | | 2 | | 1 | |
| PEG-10 Stearat | | | | 2,5 | 1 | |
| Ceteareth-20 | | | | | | 2,6 |
| Natrium Cetyl Phosphate | | | | | 2 | |
| Glyceryl Stearat, Ceteareth-12, Ceteareth-20, Cetearyl Alcohol, Cetyl Palmitat | | | | | | 5,4 |
| Stearinsäure | 3 | 2 | | | 2 | |
| Stearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | |
| Carbomer | | 0,3 | | 0,3 | 0,3 | |
| Xanthan Gum | | 0,3 | 0,4 | | | |
| C₁₂₋₁₅ Alkyl Benzoat | 5 | | | | 5 | 3 |
| 2-Phenylbenzoat | 5 | | | | | |
| Butylenglycol Dicaprylat/Dicaprat | | 5 | | 4 | | 3 |
| Dicaprylyl Ether | | 2 | | | 3 | |
| Diethylhexylnaphthalat | 3 | | | | | |
| Cyclomethicon | 2 | | 10 | 2 | | |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 5 |
| Titandioxid | 2 | 4 | | | | |
| Zinkoxid | | | | | 2 | |
| Drometrizole Trisiloxane | | | | | 3 | |
| Ethylhexylmethoxycinnamat | | 6 | 5 | | | |
| Phenylbenzimidazol Sulfonsäure | | 0,5 | 2 | | 1 | |
| Homosalat | 5 | | | 7 | | |
| Butyl Methoxydibenzoylmethan | | 3 | | | | |
| Bis-Ethylhexyloxyphenol Methoxyphenyltriazin | | 2 | 3 | | | |
| Octylsalicylat | | | | 5 | | |
| Octocrylen | | | | | 3 | |
| Farbstoff A | 0,25 | 0,5 | 0,75 | 1,0 | 1,25 | 1,5 |
| Parsol^{®} SLX | 4 | | | | | 5 |
| PVP Hexadecen Copolymer | 0,5 | | 1 | | 0,8 | |
| Coenzym Q 10 | 0,2 | 0,02 | | 0,3 | | |
| Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Pigmentfarbstoffe | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel 13: wässrige und wässrig/alkoholische Formulierungen

| | **A** | **E** | **C** | **D** | **E** | **F** |
|---|---|---|---|---|---|---|
| Ethanol | 50 | 5 | 2 | 40 | 15 | |
| Hydroxyethylcellulose | 0.5 | | | | | |
| Acrylates/C10-30 Alkyl Acrylate Crosspolymer | | | | 0,3 | 0,6 | |
| Cocoatnidopropylbetain | | | 0,3 | | | |
| UVASorb® K2A | | | | | 2 | |
| Uvinul® APlus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Farbstoff A | 0,1 | 0,25 | 0,5 | 1 | 2 | 3 |
| Farbstoff B | 3 | 2 | 1 | 0,5 | 0,25 | 0,1 |
| Farbstoff C | 0,1 | 0,25 | 0,5 | 1 | 2 | 3 |
| Farbstoff D | 3 | 2 | 1 | 0,5 | 0,25 | 0,1 |
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent^{®} 3535 | | | | 5 | | |
| Dimethicon | | | | | 3 | |
| PVP Hexadecen Copolymer | | 0,5 | | 1 | | 0.5 |
| Ethylhexyloxyglycerin | | 0,5 | | | | |
| Glycerin | 5 | 7 | 3 | 8 | | S |
| Butylenglycol | | | 5 | | 5 | |
| Metylpropandiol | | | | 4 | | |
| Vitamin E Acetat | | 0,3 | 0,2 | 0,5 | | |
| Panthenol | 0.5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0.5 |
| Trinatrium EDTA | 0,3 | 0,2 | 0,2 | 0,2 | 0,2 | 0,5 |
| Konservierungsmittel | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

### Beispiel 14: kosmetische Schäume

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20 Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| UVASorb® K2A | | | 2 |
| Uvinul® A Plus | 2 | 3 | |

| **Emulsion** | **A** | **B** | **C** |
|---|---|---|---|
| Parsol SLX^{®} | | 3 | |
| Farbstoff A | 1,0 | | |
| Farbstoff B | | 2,0 | |
| Farbstoff C | | | 1,5 |
| Farbstoff D | | | 1,5 |
| Octocrylen | 2 | | |
| Bis-Ethylhexyloxyphenol Methoxy-phenyltriazin | | 3 | |
| 2,2'-Methylen-bis-(6-(2H-benzotriazol-2-yl)-4-(1,1,3,3-tetramethylbutyl)-phenol) | | | 8 |
| 2,4,6-Tris-(biphenyl)-1,3 5-triazin | 5 | | 4 |
| C8-C16 Alkylpolyglycoside | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfum, Konservierungsmitte | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

### Beispiel 15: kosmetische Schäume

| **Emulsion** | **D** | **E** | **F** | **G** |
|---|---|---|---|---|
| Stearinsäure | 2 | | | |
| Palmitinsäure | | | 3 | 3 |
| Cetylalkohol | 2 | 2 | | |
| Cetylstearylalkohol | | | 2 | 2 |
| Stearylalkohol | | | | |
| PEG-100 Stearat | | 4 | | |
| PEG-40 Stearat | 2 | | | |
| PEG-20 Stearat | | | 3 | 3 |
| Sorbitanstearat | 0,8 | | | |
| Tridecyl Trimellitate | | 5 | | |
| C₁₂₋₁₅ Alkyl Benzoat | | | 3 | 3 |
| Butylenglycol Dicaprylat/Dicaprat | 8 | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |
| Propylenglykol | | | | |
| Glycerin | 5 | | 6 | 6 |
| NeoHeliopan^{®} AP | | 2 | | |
| Phenylbenzimidazol Sulfonsäure | 1 | | | 1 |
| Farbstoff A | 0,5 | 0,5 | 0,5 | 0,5 |
| Ethylhexylmethoxycinnamat | 5 | | 4 | 4 |
| Ethylhexyltriazon | | 2 | | 1 |
| Eusolex T-AVO^{®} | 2 | | | |
| Diethylhexylbutamidotriazon | 1 | | | |
| Butyl Methoxydibenzoylmethan | 2,5 | | 2 | 2 |
| Bis-Ethylhexyloxyphenol Methoxy-phenyltriazin | 2 | | | |
| Vitamin E Acetat | 0,2 | | 0,3 | 0,3 |
| Na₂H₂EDTA | | | | |
| Parfum, Konservierungsmitte | | | | |
| Farbstoffe, usw. | | | | |
| Natriumhydroxid | | q-s. | q.s. | |
| Triethanolamin | q.s. | | | q.s. |
| Wasser | ad 100,0 | ad 100,0 | ad 100,0 | ad 100,0 |

### Beispiel 16: (Haarfärbetest, Farbintensität)

Das Phenylazobenzoesäure-6O-L-ascorbat (PABA, Farbstoff A) wird im Vergleich zur chromophoridentischen Phenylazobenzoesäure (PABS) auf seine Haarfärbewirkung hin untersucht. Dazu werden weiße Haarsträhnen vom Büffelbauch zunächst für 60 Minuten mit Wasser inkubiert (= Versuchsvariante A). Anschließend tränkt man die Haarsträhnen mit jeweils 10%igen wässrigen Dispersionen der Farbstoffe PABA und PABS. Anschließend umwickelt man die Haarsträhnen nach Entnahme mit Aluminiumfolie und lagert für 20 Stunden bei 45°C ein. Nachfolgend werden die Haare mit Wasser gespült und gefönt. Nun werden die so gefärbten Haarsträhnen fünfmal nacheinander mit Shampoo gewaschen. Bei Behandlung mit PABA erzielt man eine intensive gelb-orangefarbene Färbung, während PABS kaum färbt.
In einem Parallelansatz (= Versuchsvariante B) inkubiert man an Stelle von Wasser mit 10%iger Ammoniaklösung und spült vor dem Färbeschritt überschüssige Ammoniaklösung mit Wasser aus.
Der jeweilige Farbeffekt wird chromametrisch gegen unbehandeltes Haar aufgenommen (Auswertung gemäß CIE-L*a*b, DIN6174). Alle Messwerte werden mit Hilfe eines Varian Cary50 Spektralphotometers in Kombination mit der Color Analysis Software Version 3.10 (228) aufgenommen. Als Remissionsmesssonde dient das Model Harrick, Barellino (Serial No BRLVA358431109019, Wellenlängenbereich 360-830nm, Intervall 1nm, Beobachtungswinkel 2°, Beleuchtung CIED65, Basislinienkorrektur gegen Weißstandard (Bariumsulfat)).
Meßwerte:

| Versuchsvariante A: | | | |
|---|---|---|---|
| | Δ L | Δ a | Δ b |
| Ascorbinsäurefarbstoff PABA | -7,72 | 6,13 | 45,1 |
| Vergleichsfarbstoff PABS | -11,7 | -0,46 | 23,6 |
| | | | |

| Versuchsvariante B: | | | |
|---|---|---|---|
| | Δ L | Δ a | Δ b |
| Ascorbinsäurefarbstoff PABA | -11,9 | 8,80 | 44,9 |
| Vergleichsfarbstoff PABS | -2,35 | -2,13 | 25,4 |

Aus den Ergebnistabellen ist ablesbar, dass für die Färbungen mit dem Ascorbinsäurefarbstoff PABA im Vergleich zum chromophoridentischen PABS sehr viel kräftigere Rot- bzw. Gelbfarbwerte erzielt werden.

## Patentansprüche

1. Verbindungen der Formel I 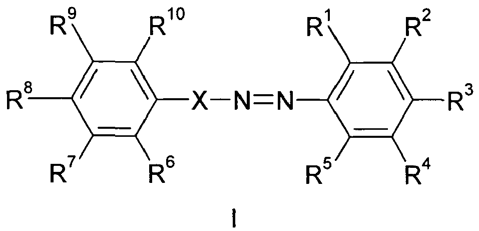 wobei X steht für eine Einfachbindung oder einen Substituenten der Formel IIa oder IIb 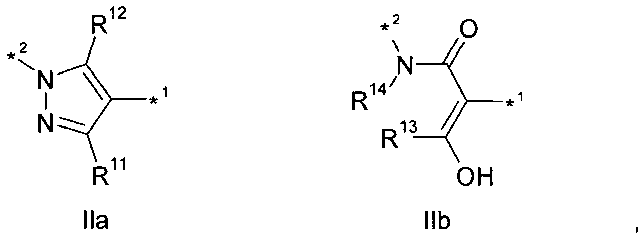 wobei *¹ zur N=N orientiert ist,
wobei genau einer der Substituenten R¹ bis R¹⁴ steht für einen Substituenten der Formel IIIa, IIIb oder IIIc 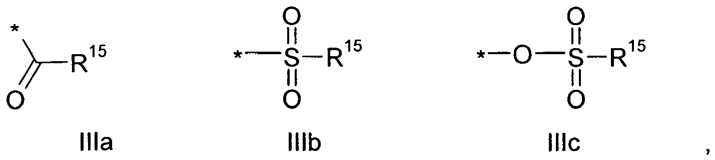 wobei R¹⁵ jeweils unabhängig voneinander steht für einen Ascorbinsäurerest der Formel IVa oder IVb oder einen Dehydroascorbinsäurerest der Formel IVc oder IVd jeweilig als L- oder D-Enantiomer 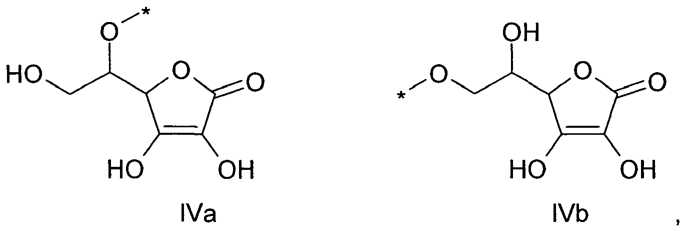 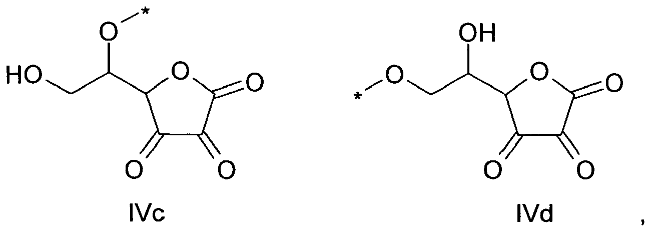 wobei die verbleibenden Substituenten R¹ bis R¹⁴ jeweils unabhängig voneinander stehen
für H, OH, NH₂, NO₂, F, Cl, Br, I, CF₃, OCF₃, O(C=O)-CH₂-NH₂, Alk¹, OAlk¹, NHAlk¹, NAlk¹₂, Cyc, (NAlk¹₃)⁺ₓAn^{x-}, (SO₂)NH₂, (SO₂)NHAlk¹, O(SO₂)OAlk¹, O(SO₂)OArl, (C=O)OAlk¹, (C=O)OArl oder Arl,
wobei Alk¹ jeweils unabhängig voneinander steht für eine geradkettige oder verzweigte C₁- bis C₂₀-Alkylgruppe oder für eine geradkettige oder verzweigte C₂- bis C₂₀-Alkenylgruppe, die mehrere Doppelbindungen aufweisen kann, wobei zumindest ein C-Atom oder mehrere nicht benachbarte C-Atome der C₁- bis C₂₀-Alkyl- oder C₂- bis C₂₀-Alkenylgruppe durch O ersetzt sein kann/können, wobei die C₁- bis C₂₀-Alkyl- oder C₂- bis C₂₀-Alkenylgruppe zumindest ein an ein primäres oder sekundäres C-Atom angebundenes OH, F, Cl, Br oder I aufweisen kann,
wobei Arl jeweils unabhängig voneinander steht für eine unsubstituierte, einfach- oder mehrfachsubstituierte C₆- bis C₂₀-Arylgruppe,
wobei Cyc steht für eine C₃- bis C₈-Cycloalkyl-Gruppe, die zumindest eine Doppelbindung aufweisen kann und/oder in der zumindest ein CH₂ durch O oder NH ersetzt sein kann,
wobei An^{x-} jeweils unabhängig voneinander steht für ein Anion der Ladung 1≤ x ≤ 3 und/oder ihrer Tautomere, Stereoisomere, einschließlich deren Mischungen in allen Verhältnissen.

2. Verbindungen nach Anspruch 1,
**dadurch gekennzeichnet, dass**
die verbleibenden Substituenten R¹ bis R¹⁴ jeweils unabhängig voneinander stehen für H, OH, NH₂, NO₂, F, Cl, Br, I, CF₃, OCF₃, Alk¹, OAlk¹, NHAlk¹, NAlk¹₂, (NAlk¹₃)⁺ₓAn^{x-}, (SO₂)NH₂, (SO₂)NHAlk¹, (C=O)OAlk¹, (C=O)OArl oder Arl.

3. Verbindungen nach Anspruch 1 oder 2,
**dadurch gekennzeichnet, dass**
die verbleibenden Substituenten R¹ bis R¹⁴ jeweils unabhängig voneinander stehen für H, OH, N(Alk¹)₂, Alk¹, N(Alk¹)₃An⁻ oder Arl.

4. Verbindungen nach einem oder mehreren der Ansprüche 1 bis 3,
**dadurch gekennzeichnet, dass**
die verbleibenden Substituenten R¹ bis R¹⁰ jeweils unabhängig voneinander stehen für H oder N(Alk¹)₂.

5. Verbindungen nach einem oder mehreren der Ansprüche 1 bis 4,
**dadurch gekennzeichnet, dass**
genau einer der Substituenten R¹ bis R⁵ oder genau einer der Substituenten R⁶ bis R¹⁰ steht für einen Substituenten der Formel IIIa, IIIb oder IIIc.

6. Verbindungen nach einem oder mehreren der Ansprüche 1 bis 5,
**dadurch gekennzeichnet, dass**
genau einer der Substituenten R¹ bis R⁵ oder genau einer der Substituenten R⁶ bis R¹⁰ steht für einen Substituenten der Formel IIIa oder
IIIb.

7. Verfahren zur Herstellung von Verbindungen der Formel I nach einem
oder mehreren der Ansprüche 1 bis 6,
bei dem in einem Veresterungsschritt genau eine Säuregruppe der Formel VIIa, VIIb oder VIIc einer Azoverbindung der Formel V, 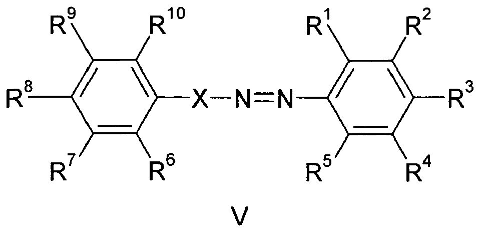 wobei X steht für eine Einfachbindung oder einen Substituenten der Formel VIa oder Vlb 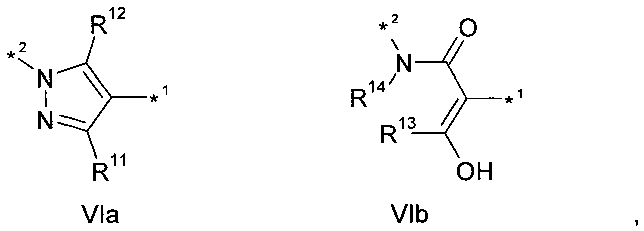 wobei *¹ zur N=N orientiert ist,
wobei genau einer der Substituenten R¹ bis R¹⁴ jeweils unabhängig voneinander steht für eine Säuregruppe der Formel VIIa, VIIb oder Vllc 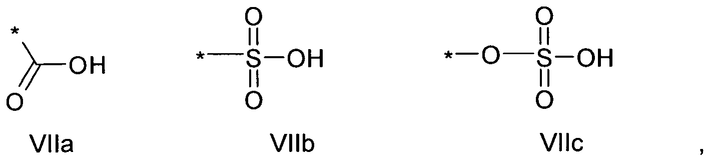 wobei die verbleibenden Substituenten R¹ bis R¹⁴ eine in den Ansprüchen 1 bis 6 beschriebene Bedeutung haben,
mit genau einer Hydroxygruppe auf der fünften oder sechsten Position der Ascorbinsäure oder der Dehydroascorbinsäure umgesetzt wird.

8. Verfahren nach Anspruch 7,
**dadurch gekennzeichnet, dass**
zusätzlich zu dem Veresterungsschritt zumindest einer der folgenden Verfahrensschritte durchgeführt werden kann:
a) ein, insbesondere vor dem Veresterungsschritt durchgeführter, Aktivierungsschritt, bei dem die Säuregruppe der Formel Vlla, Vllb oder Vllc für eine nachfolgende Veresterung aktiviert wird,
b) ein, insbesondere vor dem Veresterungsschritt oder dem Aktivierungsschritt durchgeführtes, Schützen zumindest einer Hydroxygruppe auf der fünften oder sechsten Position der Ascorbinsäure oder der Dehydroascorbinsäure mittels einer ersten Schutzgruppe,
c) ein, insbesondere vor dem Veresterungsschritt oder dem Aktivierungsschritt durchgeführtes, Schützen zumindest einer Hydroxygruppe auf der zweiten oder dritten Position der Ascorbinsäure durch eine zweite Schutzgruppe,
d) ein, insbesondere nach dem Veresterungsschritt durchgeführtes, Abspalten zumindest einer ersten oder einer zweiten Schutzgruppe, wobei die erste und die zweite Schutzgruppe unter unterschiedlichen Reaktionsbedingungen abspaltbar sind.

9. Verwendung von zumindest einer Verbindung der Formel I nach einem oder mehreren der Ansprüche 1 bis 6 und/oder deren Salz als Farbstoff für eine, insbesondere proteinhaltige, Matrix.

10. Verwendung nach Anspruch 9,
**dadurch gekennzeichnet, dass**
die Matrix Haar, Haut, Nägel, Textilien und/oder Kunststoff ist.

11. Verwendung nach einem der Ansprüche 9 oder 10,
**dadurch gekennzeichnet, dass**
zumindest zwei Verbindungen der Formel I verwendet werden, wobei die Verbindungen sich hinsichtlich R¹⁵ unterscheiden und R¹⁵ steht für einen Ascorbinsäurerest der Formel IVa oder IVb oder für einen Dehydroascorbinsäurerest der Formel IVc oder IVd jeweilig als L- oder D-Enatiomer.

12. Verfahren zum Färben einer, insbesondere proteinhaltigen, Matrix, bei dem in einem Färbeschritt direkt durch Einwirken einer Dispersion und/oder Lösung und/oder Emulsion einer Verbindung der Formel I nach einem oder mehreren der Ansprüche 1 bis 6 auf die Matrix dieselbe gefärbt wird.

13. Verfahren nach Anspruch 12,
**dadurch gekennzeichnet, dass**
die Matrix in einem Vorbehandlungsschritt zur Beeinflussung des Färbeverhaltens mittels eines Vorbehandlungsmittels vorbehandelt wird.

14. Zubereitung enthaltend zumindest eine Verbindung der Formel I nach einem oder mehreren der Ansprüche 1 bis 6.

15. Verfahren zur Herstellung einer Zubereitung nach Anspruch 14 enthaltend einen Träger, wobei zumindest eine Verbindung der Formel I mit zumindest einem für kosmetische, pharmazeutische, dermatologische Zubereitungen oder Haushaltsprodukte geeigneten Träger und gegebenenfalls Hilfsstoffen und/oder Füllstoffen gemischt, insbesondere dispergiert und/oder emulgiert und/oder gelöst, wird.

## Claims

1. Compounds of the formula I 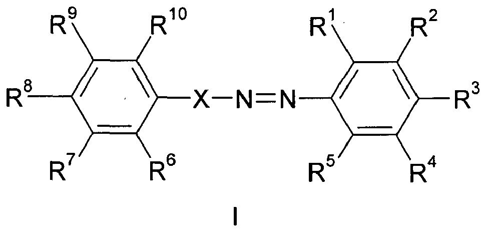 where X stands for a single bond or a substituent of the formula IIa or IIb 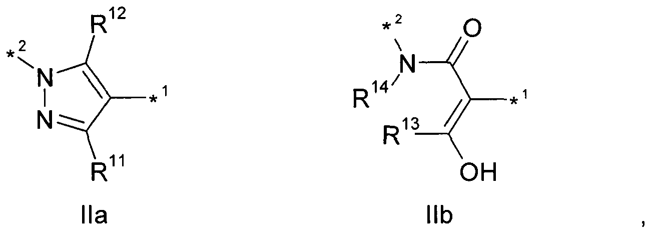 where *¹ is oriented towards the N=N,
where precisely one of the substituents R¹ to R¹⁴ stands for a substituent of the formula IIIa, IIIb or IIIc 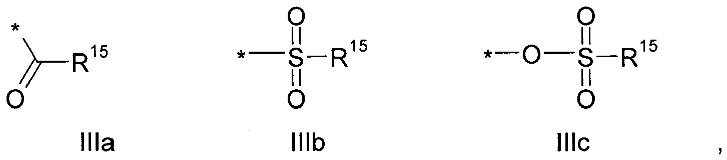 where R¹⁵ in each case stands, independently of one another, for an ascorbic acid radical of the formula IVa or IVb or a dehydroascorbic acid radical of the formula IVc or IVd, in each case as the L or D enantiomer, 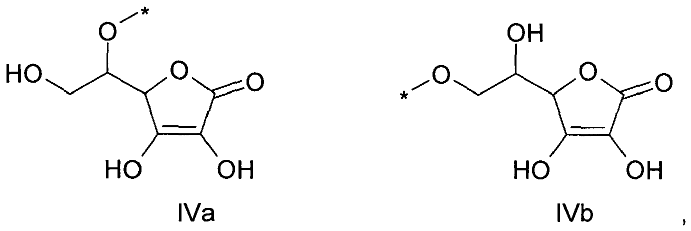 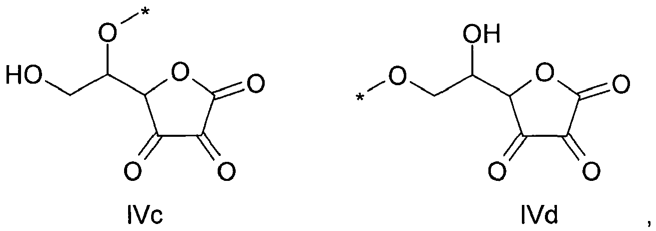 where the remaining substituents R¹ to R¹⁴ each stand, independently of one another,
for H, OH, NH₂, NO₂, F, Cl, Br, I, CF₃, OCF₃, O(C=O)-CH₂-NH₂, Alk¹, OAlk¹, NHAlk¹, NAlk¹₂, Cyc, (NAlk¹₃)⁺ₓAn^{x-}, (SO₂)NH₂, (SO₂)NHAlk¹, O(SO₂)OAlk¹, O(SO₂)OArl, (C=O)OAlk¹, (C=O)OArl or Arl, where Alk¹ in each case stands, independently of one another, for a straight-chain or branched C₁- to C₂₀-alkyl group or for a straight-chain or branched C₂- to C₂₀-alkenyl group, which may have a plurality of double bonds, where at least one C atom or a plurality of non-adjacent C atoms of the C₁- to C₂₀-alkyl or C₂- to C₂₀-alkenyl group may be replaced by O, where the C₁- to C₂₀-alkyl or C₂- to C₂₀-alkenyl group may have at least one OH, F, Cl, Br or I bonded to a primary or secondary C atom,
where Arl in each case stands, independently of one another,
for an unsubstituted, mono- or polysubstituted C₆- to C₂₀-aryl group, where Cyc stands for a C₃- to C₈-cycloalkyl group, which may have at least one double bond and/or in which at least one CH₂ may be replaced by O or NH,
where An^{x-} in each case stands, independently of one another, for an anion having the charge 1 ≤ x ≤ 3 and/or tautomers, stereoisomers thereof, including mixtures thereof in all ratios.

2. Compounds according to Claim 1,
**characterised in that**
the remaining substituents R¹ to R¹⁴ each stand, independently of one another, for H, OH, NH₂, NO₂, F, Cl, Br, I, CF₃, OCF₃, Alk¹, OAlk¹, NHAlk¹, NAlk¹₂, (NAlk¹₃)⁺ₓAn^{x-}, (SO₂)NH₂, (SO₂)NHAlk¹, (C=O)OAlk¹, (C=O)OArl or Arl.

3. Compounds according to Claim 1 or 2,
**characterised in that**
the remaining substituents R¹ to R¹⁴ each stand, independently of one another, for H, OH, N(Alk¹)₂, Alk¹, N(Alk¹)₃An⁻ or Arl.

4. Compounds according to one or more of Claims 1 to 3,
**characterised in that**
the remaining substituents R¹ to R¹⁰ each stand, independently of one another, for H or N(Alk¹)₂.

5. Compounds according to one or more of Claims 1 to 4,
**characterised in that**
precisely one of the substituents R¹ to R⁵ or precisely one of the substituents R⁶ to R¹⁰ stands for a substituent of the formula IIIa, IIIb or IIIc.

6. Compounds according to one or more of Claims 1 to 5,
**characterised in that**
precisely one of the substituents R¹ to R⁵ or precisely one of the substituents R⁶ to R¹⁰ stands for a substituent of the formula IIIa or IIIb.

7. Process for the preparation of compounds of the formula I according to one or more of Claims 1 to 6,
in which, in an esterification step, precisely one acid group of the formula VIIa, VIIb or VIIc of an azo compound of the formula V, 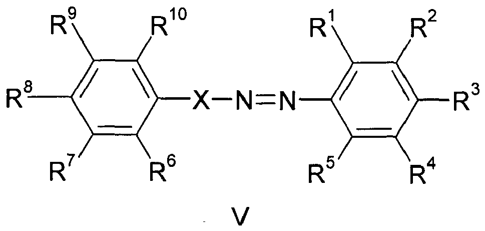 where X stands for a single bond or a substituent of the formula VIa or Vlb 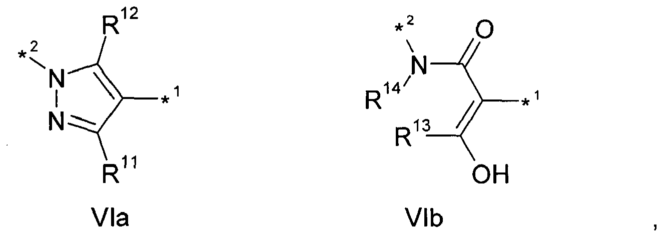 where *¹ is oriented towards the N=N,
where precisely one of the substituents R¹ to R¹⁴ in each case stands, independently of one another, for an acid group of the formula VIIa, VIIb or VIIc 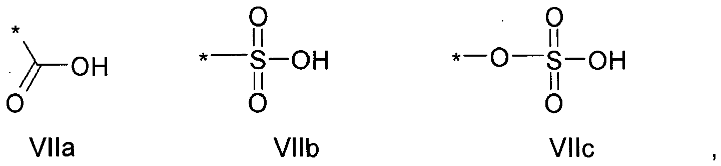 where the remaining substituents R¹ to R¹⁴ have a meaning described in Claims 1 to 6,
is reacted with precisely one hydroxyl group in the fifth or sixth position of the ascorbic acid or dehydroascorbic acid.

8. Process according to Claim 7,
**characterised in that**,
in addition to the esterification step, at least one of the following process steps can be carried out:
a) an activation step, in particular carried out before the esterification step, in which the acid group of the formula VIIa, VIIb or VIIc is activated for a subsequent esterification,
b) a protection, in particular carried out before the esterification step or activation step, of at least one hydroxyl group in the fifth or sixth position of the ascorbic acid or dehydroascorbic acid by means of a first protecting group,
c) a protection, in particular carried out before the esterification step or activation step, of at least one hydroxyl group in the second or third position of the ascorbic acid by a second protecting group,
d) a removal, in particular carried out after the esterification step, of at least one first or second protecting group, where the first and second protecting groups can be removed under different reaction conditions.

9. Use of at least one compound of the formula I according to one or more of Claims 1 to 6 and/or salt thereof as dye for a matrix, in particular a protein-containing matrix.

10. Use according to Claim 9,
**characterised in that**
the matrix is hair, skin, nails, textiles and/or plastic.

11. Use according to one of Claims 9 or 10,
**characterised in that**
at least two compounds of the formula I are used, where the compounds differ with respect to R¹⁵ and R¹⁵ stands for an ascorbic acid radical of the formula IVa or IVb or for a dehydroascorbic acid radical of the formula IVc or IVd, in each case as the L or D enantiomer.

12. Process for the colouring of a matrix, in particular a protein-containing matrix, in which the matrix is coloured directly, in a colouring step, by the action of a dispersion and/or solution and/or emulsion of a compound of the formula I according to one or more of Claims 1 to 6 on the matrix.

13. Process according to Claim 12,
**characterised in that**
the matrix is pretreated by means of a pretreatment agent in a pretreatment step in order to influence the colouring behaviour.

14. Composition comprising at least one compound of the formula I according to one or more of Claims 1 to 6.

15. Process for the preparation of a composition according to Claim 14 comprising a vehicle, in which at least one compound of the formula I is mixed, in particular dispersed and/or emulsified and/or dissolved, with at least one vehicle which is suitable for cosmetic, pharmaceutical, dermatological compositions or household products and optionally assistants and/or fillers.

## Revendications

1. Composés de la formule I 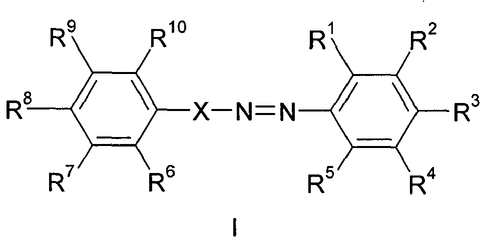 dans laquelle X représente une liaison simple ou un substituent de la formule IIa ou IIb 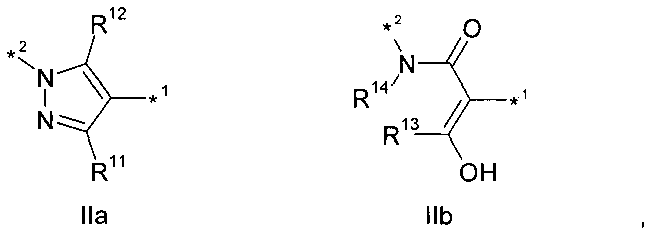 où *¹ est orienté vers N=N,
où précisément l'un des substituants R¹ à R¹⁴ représente un substituent de la formule IIIa, IIIb ou IIIc 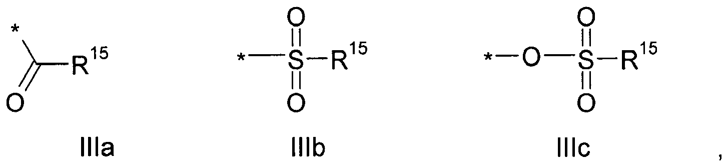 dans laquelle R¹⁵ représente, dans chaque cas indépendamment des autres cas, un radical acide ascorbique de la formule IVa ou IVb ou un radical acide déshydroascorbique de la formule IVc ou IVd, dans chaque cas en tant que l'énantiomère L ou D, 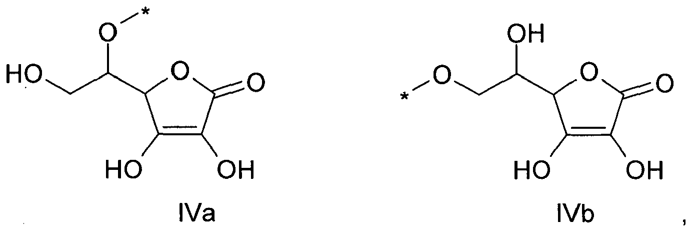 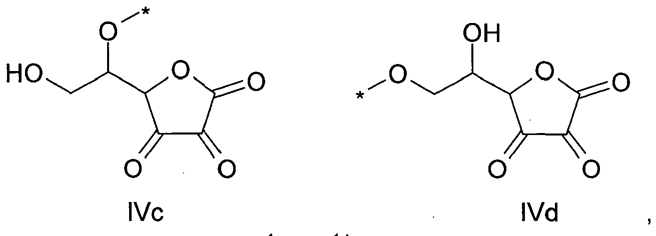 où les substituants restants R¹ à R¹⁴ représentent, chacun indépendamment des autres,
H, OH, NH₂, NO₂, F, CI, Br, I, CF₃, OCF₃, O(C=O)-CH₂-NH₂, Alk¹, OAlk¹, NHAlk¹, NAlk¹₂, Cyc, (NAlk¹₃)⁺ₓAn^{x-}, (SO₂)NH₂, (SO₂)NHAlk¹, O(SO₂)OAlk¹, O(SO₂)OArl, (C=O)OAlk¹, (C=O)OArl ou Arl,
où Alk¹ représente, dans chaque cas indépendamment des autres,
un groupe C₁- à C₂₀-alkyle en chaîne droite ou ramifié ou un groupe C₂- à C₂₀-alkényle en chaîne droite ou ramifié, lequel peut comporter une pluralité de liaisons doubles, où au moins un atome de C ou une pluralité d'atomes de C non adjacents du groupe C₁- à C₂₀-alkyle ou C₂- à C₂₀-alkényle peut/peuvent être remplacé(s) par O, où le groupe C₁- à C₂₀-alkyle ou C₂- à C₂₀-alkényle peut comporter au moins un OH, F, Cl, Br ou I lié à un atome de C primaire ou secondaire,
où Arl représente, dans chaque cas indépendamment des autres,
un groupe C₆- à C₂₀-aryle non substitué, monosubstitué ou poly-substitué,
où Cyc représente un groupe C₃- à C₈-cycloalkyle, lequel peut comporter au moins une liaison double et/ou au moins un CH₂ peut être remplacé par O ou NH,
où An^{x-} représente, dans chaque cas indépendamment des autres, un anion présentant la charge 1 ≤ x ≤ 3 et/ou des tautomères afférents, des stéréoisomères afférents, y compris des mélanges de ceux-ci selon tous rapports.

2. Composés selon la revendication 1,
**caractérisés en ce que**
les substituants restants R¹ à R¹⁴ représentent, chacun indépendamment des autres, H, OH, NH₂, NO₂, F, CI, Br, I, CF₃, OCF₃, Alk¹, OAlk¹, NHAlk¹, NAlk¹₂, (NAlk¹₃)⁺ₓAn^{x-}, (SO₂)NH₂, (SO₂)NHAlk¹, (C=O)OAlk¹, (C=O)OArl ou Arl.

3. Composés selon la revendication 1 ou 2,
**caractérisés en ce que**
les substituants restants R¹ à R¹⁴ représentent, chacun indépendamment des autres, H, OH, N(Alk¹)₂, Alk¹, N(Alk¹)₃An⁻ ou Arl.

4. Composés selon une ou plusieurs des revendications 1 à 3,
**caractérisés en ce que**
les substituants restants R¹ à R¹⁰ représentent, chacun indépendamment des autres, H ou N(Alk¹)₂.

5. Composés selon une ou plusieurs des revendications 1 à 4,
**caractérisés en ce que**
précisément l'un des substituants R¹ à R⁵ ou précisément l'un des substituants R⁶ à R¹⁰ représente un substituent de la formule IIIa, IIIb ou IIIc.

6. Composés selon une ou plusieurs des revendications 1 à 5,
**caractérisés en ce que**
précisément l'un des substituants R¹ à R⁵ ou précisément l'un des substituants R⁶ à R¹⁰ représente un substituent de la formule IIIa ou
IIIb.

7. Procédé pour la préparation de composés de la formule I selon une ou plusieurs des revendications 1 à 6,
dans lequel, au niveau d'une étape d'estérification, précisément un groupe acide de la formule VIIa, VIIb ou VIIc d'un composé azo de la formule V, 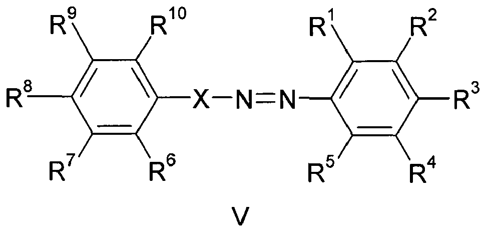 où X représente une liaison simple ou un substituent de la formule VIa ou Vlb 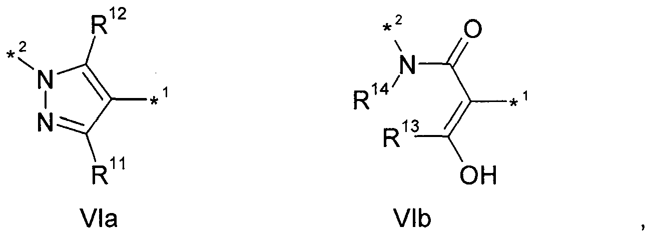 où *¹ est orienté vers N=N,
où précisément l'un des substituants R¹ à R¹⁴ représente, dans chaque cas indépendamment des autres, un groupe acide de la formule VIIa, VIIb ou VIIc 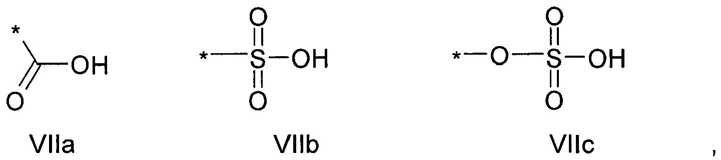 où les substituants restants R¹ à R¹⁴ présentent une signification décrite selon les revendications 1 à 6,
est amené à réagir avec précisément un groupe hydroxyle à la cinquième ou sixième position de l'acide ascorbique ou de l'acide déshydroascorbique.

8. Procédé selon la revendication 7,
**caractérisé en ce que**,
en plus de l'étape d'estérification, au moins l'une des étapes de procédé qui suivent peut être mise en oeuvre :
a) une étape d'activation, en particulier mise en oeuvre avant l'étape d'estérification, au niveau de laquelle le groupe acide de la formule VIIa, VIIb ou VIIc est activé pour une estérification qui suit,
b) une étape de protection, en particulier mise en oeuvre avant l'étape d'estérification ou l'étape d'activation, d'au moins un groupe hydroxyle à la cinquième ou sixième position de l'acide ascorbique ou de l'acide déshydroascorbique au moyen d'un premier groupe de protection,
c) une étape de protection, en particulier mise en oeuvre avant l'étape d'estérification ou l'étape d'activation, d'au moins un groupe hydroxyle à la deuxième ou troisième position de l'acide ascorbique au moyen d'un second groupe de protection,
d) une étape d'enlèvement, en particulier mise en oeuvre après l'étape d'estérification, d'au moins un premier ou second groupe de protection, où les premier et second groupes de protection peuvent être enlevés sous des conditions de réaction différentes.

9. Utilisation d'au moins un composé de la formule I selon une ou plusieurs des revendications 1 à 6 et/ou de son sel en tant que colorant pour une matrice, en particulier une matrice contenant une protéine.

10. Utilisation selon la revendication 9,
**caractérisée en ce que**
la matrice est constituée par des cheveux, de la peau, des ongles, des textiles et/ou une matière plastique.

11. Utilisation selon l'une des revendications 9 ou 10,
**caractérisée en ce que**
au moins deux composés de la formule I sont utilisés, où les composés diffèrent en ce qui concerne R¹⁵ et R¹⁵ représente un radical acide ascorbique de la formule IVa ou IVb ou un radical acide déshydroascorbique de la formule IVc ou IVd, dans chaque cas en tant que l'énantiomère L ou D.

12. Procédé pour la coloration d'une matrice, en particulier une matrice contenant des protéines, dans lequel la matrice est colorée directement, au niveau d'une étape de coloration, par l'action d'une dispersion et/ou d'une solution et/ou d'une émulsion d'un composé de la formule I selon une ou plusieurs des revendications 1 à 6 sur la matrice.

13. Procédé selon la revendication 12,
**caractérisé en ce que**
la matrice est prétraitée au moyen d'un agent de prétraitement au niveau d'une étape de prétraitement afin d'influencer le comportement en coloration.

14. Composition comprenant au moins un composé de la formule I selon une ou plusieurs des revendications 1 à 6.

15. Procédé pour la préparation d'une composition selon la revendication 14 comprenant un véhicule, dans lequel au moins un composé de la formule I est mélangé, en particulier est dispersé et/ou émulsifié et/ou dissout, avec au moins un véhicule qui convient pour des compositions cosmétiques, pharmaceutiques, dermatologiques ou des produits d'entretien et en option, des agents favorisants et/ou des agents de remplissage.
